# EUROPÄISCHE PATENTSCHRIFT

(11) **EP 3 194 588 B1**
(45) Veröffentlichungstag und Bekanntmachung des Hinweises auf die Patenterteilung: **21.10.2020**
(21) Anmeldenummer: 15775080.3
(22) Anmeldetag: 27.08.2015
(51) Int. Cl.: C12P 7/26, C12N 9/88

(54) **LYASE SOWIE VERFAHREN ZUR ASYMMETRISCHEN SYNTHESE VON (S)-PHENYLACETYLCARBINOL**
LYASE AND METHOD FOR ASYMMETRIC SYNTHESIS OF (S)-PHENYLACETYLCARBINOL
LYASE ET PROCEDE DE SYNTHÈSE ASYMÉTRIQUE DE (S)-PHÉNYLACÉTYLCARBINOL

(30) Priorität: 16.09.2014 DE 102014013642
(43) Veröffentlichungstag der Anmeldung: 26.07.2017
(73) Patentinhaber: Forschungszentrum Jülich GmbH, 52425 Jülich (DE)
(72) Erfinder: ROTHER, Dörte, 50354 Hürth (DE); POHL, Martina, 52066 Aachen (DE); SEHL, Torsten, 77654 Offenburg (DE); MARX, Lisa, 64342 Seeheim-Jugenheim (DE); WESTPHAL, Robert, 04155 Leipzig (DE)
(86) Internationale Anmeldenummer: PCT/DE2015/000435
(87) Internationale Veröffentlichungsnummer: WO 2016/041535

(56) Entgegenhaltungen:
- WESTPHAL ROBERT ET AL: "A tailor-made chimeric thiamine diphosphate dependent enzyme for the direct asymmetric synthesis of (S)-benzoins", ANGEWANDTE CHEMIE (INTERNATIONAL EDITION), WILEY - VCH VERLAG GMBH & CO, DE, Bd. 53, Nr. 35, 25. August 2014 (2014-08-25), Seiten 9376-9379, XP009187357, ISSN: 1521-3773
- DÖRTE ROTHER NEÉ GOCKE ET AL: "S-Selective Mixed Carboligation by Structure-Based Design of the Pyruvate Decarboxylase from Acetobacter pasteurianus", CHEMCATCHEM, Bd. 3, Nr. 10, 31. August 2011 (2011-08-31), Seiten 1587-1596, XP55134569, ISSN: 1867-3880, DOI: 10.1002/cctc.201100054
- TINA GERHARDS ET AL: "Influence of Organic Solvents on Enzymatic Asymmetric Carboligations", ADVANCED SYNTHESIS & CATALYSIS, Bd. 354, Nr. 14-15, 4. Oktober 2012 (2012-10-04), Seiten 2805-2820, XP055134571, ISSN: 1615-4150, DOI: 10.1002/adsc.201200284
- TORSTEN SEHL ET AL: "Efficient 2-step biocatalytic strategies for the synthesis of all nor(pseudo)ephedrine isomers", GREEN CHEMISTRY, Bd. 16, Nr. 6, 1. Januar 2014 (2014-01-01) , Seite 3341, XP055124877, ISSN: 1463-9262, DOI: 10.1039/c4gc00100a
- TORSTEN SEHL ET AL: "Asymmetric synthesis of (S)-phenylacetylcarbinol - closing a gap in C-C bon formation", JOURNAL OF IMMUNOTHERAPY, LIPPINCOTT WILLIAMS & WILKINS, US, vol. 19, 26 January 2017 (2017-01-26), pages 380-384, XP009508211, ISSN: 1463-9262, DOI: 10.1039/c6gc01803c

### SEQUENCE LISTING

<110> Forschungszentrum Jülich GmbH
<120> Lyase sowie verfahren zur asymmetrischen Synthese von Phenylacetylcarbinol
<130> PT1.2668
<160> 53
<170> PatentIn version 3.5
<210> 1
   <211> 560
   <212> PRT
   <213> artificial
<220>
   <223> künstlich hergestellt
<400> 1
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Gly Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Trp Gly
   530 535 540
   Arg Lys Val Ala Ser Thr Asn Ala Arg Lys Thr Thr Leu Ala Leu Glu
   545 550 555 560
<210> 2
   <211> 560
   <212> PRT
   <213> artificial
<220>
   <223> künstlich hergestellt
<400> 2
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
                   245                 250                 255
   Phe Arg Gly Leu Tyr Trp Gly Glu Val Ser Asn Pro Gly Val Gln Glu
               260                 265                 270
   Leu Val Glu Thr Ser Asp Ala Leu Leu Cys Ile Ala Pro Val Phe Asn
           275                 280                 285
   Asp Tyr Ser Thr Val Gly Trp Ser Ala Trp Pro Lys Gly Pro Asn Val
       290                 295                 300
   Ile Leu Ala Glu Pro Asp Arg Val Thr Val Asp Gly Arg Ala Tyr Asp
   305                 310                 315                 320
   Gly Phe Thr Leu Arg Ala Phe Leu Gln Ala Leu Ala Glu Lys Ala Pro
                   325                 330                 335
   Ala Arg Pro Ala Ser Ala Gln Lys Ser Ser Val Pro Thr Cys Ser Leu
               340                 345                 350
   Thr Ala Thr Ser Asp Glu Ala Gly Leu Thr Asn Asp Glu Ile Val Arg
           355                 360                 365
   His Ile Asn Ala Leu Leu Thr Ser Asn Thr Thr Leu Val Ala Glu Thr
       370                 375                 380
   Gly Asp Ser Trp Phe Asn Ala Met Arg Met Thr Leu Pro Arg Gly Ala
   385                 390                 395                 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Ala Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Trp Gly
   530 535 540
   Arg Lys Val Ala Ser Thr Asn Ala Arg Lys Thr Thr Leu Ala Leu Glu
   545 550 555 560
<210> 3
   <211> 560
   <212> PRT
   <213> artificial
<220>
   <223> künstlich hergestellt
<400> 3
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65 70 75 80
   Met Asn Ala Leu Gly Gly Ala Tyr Ala Glu Asn Leu Pro Val Ile Leu
   85 90 95
   Ile Ser Gly Ala Pro Asn Ser Asn Asp Gln Gly Thr Gly His Ile Leu
   100 105 110
   His His Thr Ile Gly Lys Thr Asp Tyr Ser Tyr Gln Leu Glu Met Ala
   115 120 125
   Arg Gln Val Thr Cys Ala Ala Glu Ser Ile Thr Asp Ala His Ser Ala
   130 135 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Val Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Trp Gly
   530 535 540
   Arg Lys Val Ala Ser Thr Asn Ala Arg Lys Thr Thr Leu Ala Leu Glu
   545 550 555 560
<210> 4
   <211> 1680
   <212> DNA
   <213> artificial
<220>
   <223> künstlich hergestellt
<400> 4
   atgacctata ctgttggcat gtatcttgca gaacgccttg tacagatcgg gctgaagcat 60
   cacttcgccg tggcgggcga ctacaatctc gttcttctgg atcagttgct cctcaacaag 120
   gacatgaaac agatctattg ctgcaatgag ttgaactgtg gcttcagcgc ggaaggctac 180
   gcccgttcta acggggctgc ggcagcggtt gtcaccttca gcgttggcgc catttccgcc 240
   atgaacgccc tcggcggcgc ctatgccgaa aacctgccgg ttatcctgat ttccggcgcg 300
   cccaacagca atgatcaggg cacaggtcat atcctgcatc acacaatcgg caagacggat 360
   tacagctacc agcttgaaat ggcccgtcag gtcacctgtg ccgccgaaag cattaccgac 420
   gctcactccg ccccggccaa gattgaccac gtcattcgca cggcgctgcg cgagcgtaag 480
   ccggcctatc tggacatcgc gtgcaacatt gcctccgagc cctgcgtgcg gcctggccct 540
   gtcagcagcc tgctgtccga gcctgaaatc gaccacacga gcctgaaggc cgcagtggac 600
   gccacggttg ccttgctgga aaaatcggcc agccccgtca tgctgctggg cagcaagctg 660
   cgggccgcca acgcactggc cgcaaccgaa acgctggcag acaagctgca atgcgcggtg 720
   accatcatgg cggccgcgaa aggctttttc cccgaagacc acgcgggttt ccgcggcctg 780
   tactggggcg aagtctcgaa ccccggcgtg caggaactgg tggagacctc cgacgcactg 840
   ctgtgcatcg cccccgtatt caacgactat tcaacagtcg gctggtcggc atggcccaag 900
   ggccccaatg tgattctggc tgagcccgac cgcgtaacgg tcgatggccg cgcctatgac 960
   ggctttaccc tgcgcgcctt cctgcaggct ctggcggaaa aagcccccgc gcgcccggcc 1020
   tccgcacaga aaagcagcgt cccgacgtgc tcgctcaccg cgacatccga tgaagccggt 1080
   ctgacgaatg acgaaatcgt ccgtcatatc aacgccctgc tgacatcaaa cacgacgctg 1140
   gtggcagaaa ccggcgattc atggttcaat gccatgcgca tgaccctgcc gcgcggtgcg 1200
   cgcgtggaac tggaaatgca gtggggccat atcggctggt ccgtgccctc cgccttcggc 1260
   aatgccatgg gctcgcagga ccgccagcat gtggtgatgg taggcgatgg ctccttccag 1320
   cttaccgcgc aggaagtggc tcagatggtg cgctacgaac tgcccgtcat tatctttctg 1380
   atcaacaacc gtggctatgt cggtggcatc gccattcatg acggcccgta caactatatc 1440
   aagaactggg attacgccgg cctgatggaa gtcttcaacg ccggagaagg ccatggactt 1500
   ggcctgaaag ccaccacccc gaaggaactg acagaagcca tcgccagggc aaaagccaat 1560
   acccgcggcc cgacgctgat cgaatgccag atcgaccgca cggactgcac ggatatgctg 1620
   gttcaatggg gccgcaaggt tgcctcaacc aacgcgcgca agaccactct ggccctcgag 1680
<210> 5
   <211> 7041
   <212> DNA
   <213> artificial
<220>
   <223> künstlich hergestellt
<400> 5
   tggcgaatgg gacgcgccct gtagcggcgc attaagcgcg gcgggtgtgg tggttacgcg        60
   cagcgtgacc gctacacttg ccagcgccct agcgcccgct cctttcgctt tcttcccttc       120
   ctttctcgcc acgttcgccg gctttccccg tcaagctcta aatcgggggc tccctttagg       180
   gttccgattt agtgctttac ggcacctcga ccccaaaaaa cttgattagg gtgatggttc 240
   acgtagtggg ccatcgccct gatagacggt ttttcgccct ttgacgttgg agtccacgtt 300
   ctttaatagt ggactcttgt tccaaactgg aacaacactc aaccctatct cggtctattc 360
   ttttgattta taagggattt tgccgatttc ggcctattgg ttaaaaaatg agctgattta 420
   acaaaaattt aacgcgaatt ttaacaaaat attaacgttt acaatttcag gtggcacttt 480
   tcggggaaat gtgcgcggaa cccctatttg tttatttttc taaatacatt caaatatgta 540
   tccgctcatg agacaataac cctgataaat gcttcaataa tattgaaaaa ggaagagtat 600
   gagtattcaa catttccgtg tcgcccttat tccctttttt gcggcatttt gccttcctgt 660
   ttttgctcac ccagaaacgc tggtgaaagt aaaagatgct gaagatcagt tgggtgcacg 720
   agtgggttac atcgaactgg atctcaacag cggtaagatc cttgagagtt ttcgccccga 780
   agaacgtttt ccaatgatga gcacttttaa agttctgcta tgtggcgcgg tattatcccg 840
   tattgacgcc gggcaagagc aactcggtcg ccgcatacac tattctcaga atgacttggt 900
   tgagtactca ccagtcacag aaaagcatct tacggatggc atgacagtaa gagaattatg 960
   cagtgctgcc ataaccatga gtgataacac tgcggccaac ttacttctga caacgatcgg 1020
   aggaccgaag gagctaaccg cttttttgca caacatgggg gatcatgtaa ctcgccttga 1080
   tcgttgggaa ccggagctga atgaagccat accaaacgac gagcgtgaca ccacgatgcc 1140
   tgcagcaatg gcaacaacgt tgcgcaaact attaactggc gaactactta ctctagcttc 1200
   ccggcaacaa ttaatagact ggatggaggc ggataaagtt gcaggaccac ttctgcgctc 1260
   ggcccttccg gctggctggt ttattgctga taaatctgga gccggtgagc gtgggtctcg 1320
   cggtatcatt gcagcactgg ggccagatgg taagccctcc cgtatcgtag ttatctacac 1380
   gacggggagt caggcaacta tggatgaacg aaatagacag atcgctgaga taggtgcctc 1440
   actgattaag cattggtaac tgtcagacca agtttactca tatatacttt agattgattt 1500
   aaaacttcat ttttaattta aaaggatcta ggtgaagatc ctttttgata atctcatgac 1560
   caaaatccct taacgtgagt tttcgttcca ctgagcgtca gaccccgtag aaaagatcaa 1620
   aggatcttct tgagatcctt tttttctgcg cgtaatctgc tgcttgcaaa caaaaaaacc 1680
   accgctacca gcggtggttt gtttgccgga tcaagagcta ccaactcttt ttccgaaggt 1740
   aactggcttc agcagagcgc agataccaaa tactgtcctt ctagtgtagc cgtagttagg 1800
   ccaccacttc aagaactctg tagcaccgcc tacatacctc gctctgctaa tcctgttacc 1860
   agtggctgct gccagtggcg ataagtcgtg tcttaccggg ttggactcaa gacgatagtt 1920
   accggataag gcgcagcggt cgggctgaac ggggggttcg tgcacacagc ccagcttgga 1980
   gcgaacgacc tacaccgaac tgagatacct acagcgtgag ctatgagaaa gcgccacgct 2040
   tcccgaaggg agaaaggcgg acaggtatcc ggtaagcggc agggtcggaa caggagagcg 2100
   cacgagggag cttccagggg gaaacgcctg gtatctttat agtcctgtcg ggtttcgcca 2160
   cctctgactt gagcgtcgat ttttgtgatg ctcgtcaggg gggcggagcc tatggaaaaa 2220
   cgccagcaac gcggcctttt tacggttcct ggccttttgc tggccttttg ctcacatgtt 2280
   ctttcctgcg ttatcccctg attctgtgga taaccgtatt accgcctttg agtgagctga 2340
   taccgctcgc cgcagccgaa cgaccgagcg cagcgagtca gtgagcgagg aagcggaaga 2400
   gcgcctgatg cggtattttc tccttacgca tctgtgcggt atttcacacc gcatatatgg 2460
   tgcactctca gtacaatctg ctctgatgcc gcatagttaa gccagtatac actccgctat 2520
   cgctacgtga ctgggtcatg gctgcgcccc gacacccgcc aacacccgct gacgcgccct 2580
   gacgggcttg tctgctcccg gcatccgctt acagacaagc tgtgaccgtc tccgggagct 2640
   gcatgtgtca gaggttttca ccgtcatcac cgaaacgcgc gaggcagctg cggtaaagct 2700
   catcagcgtg gtcgtgaagc gattcacaga tgtctgcctg ttcatccgcg tccagctcgt 2760
   tgagtttctc cagaagcgtt aatgtctggc ttctgataaa gcgggccatg ttaagggcgg 2820
   ttttttcctg tttggtcact gatgcctccg tgtaaggggg atttctgttc atgggggtaa 2880
   tgataccgat gaaacgagag aggatgctca cgatacgggt tactgatgat gaacatgccc 2940
   ggttactgga acgttgtgag ggtaaacaac tggcggtatg gatgcggcgg gaccagagaa 3000
   aaatcactca gggtcaatgc cagcgcttcg ttaatacaga tgtaggtgtt ccacagggta 3060
   gccagcagca tcctgcgatg cagatccgga acataatggt gcagggcgct gacttccgcg 3120
   tttccagact ttacgaaaca cggaaaccga agaccattca tgttgttgct caggtcgcag 3180
   acgttttgca gcagcagtcg cttcacgttc gctcgcgtat cggtgattca ttctgctaac 3240
   cagtaaggca accccgccag cctagccggg tcctcaacga caggagcacg atcatgcgca 3300
   cccgtggggc cgccatgccg gcgataatgg cctgcttctc gccgaaacgt ttggtggcgg 3360
   gaccagtgac gaaggcttga gcgagggcgt gcaagattcc gaataccgca agcgacaggc 3420
   cgatcatcgt cgcgctccag cgaaagcggt cctcgccgaa aatgacccag agcgctgccg 3480
   gcacctgtcc tacgagttgc atgataaaga agacagtcat aagtgcggcg acgatagtca 3540
   tgccccgcgc ccaccggaag gagctgactg ggttgaaggc tctcaagggc atcggtcgag 3600
   atcccggtgc ctaatgagtg agctaactta cattaattgc gttgcgctca ctgcccgctt 3660
   tccagtcggg aaacctgtcg tgccagctgc attaatgaat cggccaacgc gcggggagag 3720
   gcggtttgcg tattgggcgc cagggtggtt tttcttttca ccagtgagac gggcaacagc 3780
   tgattgccct tcaccgcctg gccctgagag agttgcagca agcggtccac gctggtttgc 3840
   cccagcaggc gaaaatcctg tttgatggtg gttaacggcg ggatataaca tgagctgtct 3900
   tcggtatcgt cgtatcccac taccgagata tccgcaccaa cgcgcagccc ggactcggta 3960
   atggcgcgca ttgcgcccag cgccatctga tcgttggcaa ccagcatcgc agtgggaacg 4020
   atgccctcat tcagcatttg catggtttgt tgaaaaccgg acatggcact ccagtcgcct 4080
   tcccgttccg ctatcggctg aatttgattg cgagtgagat atttatgcca gccagccaga 4140
   cgcagacgcg ccgagacaga acttaatggg cccgctaaca gcgcgatttg ctggtgaccc 4200
   aatgcgacca gatgctccac gcccagtcgc gtaccgtctt catgggagaa aataatactg 4260
   ttgatgggtg tctggtcaga gacatcaaga aataacgccg gaacattagt gcaggcagct 4320
   tccacagcaa tggcatcctg gtcatccagc ggatagttaa tgatcagccc actgacgcgt 4380
   tgcgcgagaa gattgtgcac cgccgcttta caggcttcga cgccgcttcg ttctaccatc 4440
   gacaccacca cgctggcacc cagttgatcg gcgcgagatt taatcgccgc gacaatttgc 4500
   gacggcgcgt gcagggccag actggaggtg gcaacgccaa tcagcaacga ctgtttgccc 4560
   gccagttgtt gtgccacgcg gttgggaatg taattcagct ccgccatcgc cgcttccact 4620
   ttttcccgcg ttttcgcaga aacgtggctg gcctggttca ccacgcggga aacggtctga 4680
   taagagacac cggcatactc tgcgacatcg tataacgtta ctggtttcac attcaccacc 4740
   ctgaattgac tctcttccgg gcgctatcat gccataccgc gaaaggtttt gcgccattcg 4800
   atggtgtccg ggatctcgac gctctccctt atgcgactcc tgcattagga agcagcccag 4860
   tagtaggttg aggccgttga gcaccgccgc cgcaaggaat ggtgcatgca aggagatggc 4920
   gcccaacagt cccccggcca cggggcctgc caccataccc acgccgaaac aagcgctcat 4980
   gagcccgaag tggcgagccc gatcttcccc atcggtgatg tcggcgatat aggcgccagc 5040
   aaccgcacct gtggcgccgg tgatgccggc cacgatgcgt ccggcgtaga ggatcgagat 5100
   ctcgatcccg cgaaattaat acgactcact ataggggaat tgtgagcgga taacaattcc 5160
   cctctagaaa taattttgtt taactttaag aaggagatat acatatgacc tatactgttg 5220
   gcatgtatct tgcagaacgc cttgtacaga tcgggctgaa gcatcacttc gccgtggcgg 5280
   gcgactacaa tctcgttctt ctggatcagt tgctcctcaa caaggacatg aaacagatct 5340
   attgctgcaa tgagttgaac tgtggcttca gcgcggaagg ctacgcccgt tctaacgggg 5400
   ctgcggcagc ggttgtcacc ttcagcgttg gcgccatttc cgccatgaac gccctcggcg 5460
   gcgcctatgc cgaaaacctg ccggttatcc tgatttccgg cgcgcccaac agcaatgatc 5520
   agggcacagg tcatatcctg catcacacaa tcggcaagac ggattacagc taccagcttg 5580
   aaatggcccg tcaggtcacc tgtgccgccg aaagcattac cgacgctcac tccgccccgg 5640
   ccaagattga ccacgtcatt cgcacggcgc tgcgcgagcg taagccggcc tatctggaca 5700
   tcgcgtgcaa cattgcctcc gagccctgcg tgcggcctgg ccctgtcagc agcctgctgt 5760
   ccgagcctga aatcgaccac acgagcctga aggccgcagt ggacgccacg gttgccttgc 5820
   tggaaaaatc ggccagcccc gtcatgctgc tgggcagcaa gctgcgggcc gccaacgcac 5880
   tggccgcaac cgaaacgctg gcagacaagc tgcaatgcgc ggtgaccatc atggcggccg 5940
   cgaaaggctt tttccccgaa gaccacgcgg gtttccgcgg cctgtactgg ggcgaagtct 6000
   cgaaccccgg cgtgcaggaa ctggtggaga cctccgacgc actgctgtgc atcgcccccg 6060
   tattcaacga ctattcaaca gtcggctggt cggcatggcc caagggcccc aatgtgattc 6120
   tggctgagcc cgaccgcgta acggtcgatg gccgcgccta tgacggcttt accctgcgcg 6180
   ccttcctgca ggctctggcg gaaaaagccc ccgcgcgccc ggcctccgca cagaaaagca 6240
   gcgtcccgac gtgctcgctc accgcgacat ccgatgaagc cggtctgacg aatgacgaaa 6300
   tcgtccgtca tatcaacgcc ctgctgacat caaacacgac gctggtggca gaaaccggcg 6360
   attcatggtt caatgccatg cgcatgaccc tgccgcgcgg tgcgcgcgtg gaactggaaa 6420
   tgcagtgggg ccatatcggc tggtccgtgc cctccgcctt cggcaatgcc atgggctcgc 6480
   aggaccgcca gcatgtggtg atggtaggcg atggctcctt ccagcttacc gcgcaggaag 6540
   tggctcagat ggtgcgctac gaactgcccg tcattatctt tctgatcaac aaccgtggct 6600
   atgtcggtgg catcgccatt catgacggcc cgtacaacta tatcaagaac tgggattacg 6660
   ccggcctgat ggaagtcttc aacgccggag aaggccatgg acttggcctg aaagccacca 6720
   ccccgaagga actgacagaa gccatcgcca gggcaaaagc caatacccgc ggcccgacgc 6780
   tgatcgaatg ccagatcgac cgcacggact gcacggatat gctggttcaa tggggccgca 6840
   aggttgcctc aaccaacgcg cgcaagacca ctctggccct cgagcaccac caccaccacc 6900
   actgagatcc ggctgctaac aaagcccgaa aggaagctga gttggctgct gccaccgctg 6960
   agcaataact agcataaccc cttggggcct ctaaacgggt cttgaggggt tttttgctga 7020
   aaggaggaac tatatccgga t 7041
<210> 6
   <211> 29
   <212> DNA
   <213> artificial
<220>
   <223> künstlich hergestellt
<220>
   <221> misc_feature
   <222> (14)..(14)
   <223> n is a, c, g, or t
<400> 6
   ccgtggctat gtcndtggca tcgccattc 29
<210> 7
   <211> 29
   <212> DNA
   <213> artificial
<220>
   <223> künstlich hergestellt
<220>
   <221> misc_feature
   <222> (16)..(16)
   <223> n is a, c, g, or t
<400> 7
   gaatggcgat gccahngaca tagccacgg 29
<210> 8
   <211> 1680
   <212> DNA
   <213> artificial
<220>
   <223> künstlich hergestellt
<400> 8
   atgacctata ctgttggcat gtatcttgca gaacgccttg tacagatcgg gctgaagcat      60
   cacttcgccg tggcgggcga ctacaatctc gttcttctgg atcagttgct cctcaacaag     120
   gacatgaaac agatctattg ctgcaatgag ttgaactgtg gcttcagcgc ggaaggctac     180
   gcccgttcta acggggctgc ggcagcggtt gtcaccttca gcgttggcgc catttccgcc     240
   atgaacgccc tcggcggcgc ctatgccgaa aacctgccgg ttatcctgat ttccggcgcg     300
   cccaacagca atgatcaggg cacaggtcat atcctgcatc acacaatcgg caagacggat     360
   tacagctacc agcttgaaat ggcccgtcag gtcacctgtg ccgccgaaag cattaccgac     420
   gctcactccg ccccggccaa gattgaccac gtcattcgca cggcgctgcg cgagcgtaag     480
   ccggcctatc tggacatcgc gtgcaacatt gcctccgagc cctgcgtgcg gcctggccct     540
   gtcagcagcc tgctgtccga gcctgaaatc gaccacacga gcctgaaggc cgcagtggac     600
   gccacggttg ccttgctgga aaaatcggcc agccccgtca tgctgctggg cagcaagctg     660
   cgggccgcca acgcactggc cgcaaccgaa acgctggcag acaagctgca atgcgcggtg     720
   accatcatgg cggccgcgaa aggctttttc cccgaagacc acgcgggttt ccgcggcctg     780
   tactggggcg aagtctcgaa ccccggcgtg caggaactgg tggagacctc cgacgcactg     840
   ctgtgcatcg cccccgtatt caacgactat tcaacagtcg gctggtcggc atggcccaag     900
   ggccccaatg tgattctggc tgagcccgac cgcgtaacgg tcgatggccg cgcctatgac     960
   ggctttaccc tgcgcgcctt cctgcaggct ctggcggaaa aagcccccgc gcgcccggcc    1020
   tccgcacaga aaagcagcgt cccgacgtgc tcgctcaccg cgacatccga tgaagccggt    1080
   ctgacgaatg acgaaatcgt ccgtcatatc aacgccctgc tgacatcaaa cacgacgctg    1140
   gtggcagaaa ccggcgattc atggttcaat gccatgcgca tgaccctgcc gcgcggtgcg    1200
   cgcgtggaac tggaaatgca gtggggccat atcggctggt ccgtgccctc cgccttcggc    1260
   aatgccatgg gctcgcagga ccgccagcat gtggtgatgg taggcgatgg ctccttccag    1320
   cttaccgcgc aggaagtggc tcagatggtg cgctacgaac tgcccgtcat tatctttctg    1380
   atcaacaacc gtggctatgt cattggcatc gccattcatg acggcccgta caactatatc    1440
   aagaactggg attacgccgg cctgatggaa gtcttcaacg ccggagaagg ccatggactt    1500
   ggcctgaaag ccaccacccc gaaggaactg acagaagcca tcgccagggc aaaagccaat    1560
   acccgcggcc cgacgctgat cgaatgccag atcgaccgca cggactgcac ggatatgctg    1620
   gttcaatggg gccgcaaggt tgcctcaacc aacgcgcgca agaccactct ggccctcgag    1680
<210> 9
   <211> 560
   <212> PRT
   <213> artificial
<220>
   <223> künstlich hergestellt
<400> 9
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
                   245                 250                 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Gly Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln His Gly
   530 535 540
   Arg Lys Val Ala Ser Thr Asn Ala Arg Lys Thr Thr Leu Ala Leu Glu
   545 550 555 560
<210> 10
   <211> 560
   <212> PRT
   <213> artificial
<220>
   <223> künstlich hergestellt
<400> 10
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Gly Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
                   485                 490                 495
   Gly His Gly Leu Gly Leu Lys Ala Thr Thr Pro Lys Glu Leu Thr Glu
               500                 505                 510
   Ala Ile Ala Arg Ala Lys Ala Asn Thr Arg Gly Pro Thr Leu Ile Glu
           515                 520                 525
   Cys Gln Ile Asp Arg Thr Asp Cys Thr Asp Met Leu Val Gln Phe Gly
       530                 535                 540
   Arg Lys Val Ala Ser Thr Asn Ala Arg Lys Thr Thr Leu Ala Leu Glu
   545                 550                 555                 560
<210> 11
   <211> 560
   <212> PRT
   <213> artificial
<220>
   <223> künstlich hergestellt
<400> 11
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                      80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Gly Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Pro Gly
   530 535 540
   Arg Lys Val Ala Ser Thr Asn Ala Arg Lys Thr Thr Leu Ala Leu Glu
   545 550 555 560
<210> 12
   <211> 560
   <212> PRT
   <213> artificial
<220>
   <223> künstlich hergestellt
<400> 12
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
   100 105 110
   His His Thr Ile Gly Lys Thr Asp Tyr Ser Tyr Gln Leu Glu Met Ala
   115 120 125
   Arg Gln Val Thr Cys Ala Ala Glu Ser Ile Thr Asp Ala His Ser Ala
   130 135 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Gly Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Ile Gly
   530 535 540
   Arg Lys Val Ala Ser Thr Asn Ala Arg Lys Thr Thr Leu Ala Leu Glu
   545 550 555 560
<210> 13
   <211> 560
   <212> PRT
   <213> artificial
<220>
   <223> künstlich hergestellt
<400> 13
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
               35                      40                      45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
                   245                 250                 255
   Phe Arg Gly Leu Tyr Trp Gly Glu Val Ser Asn Pro Gly Val Gln Glu
               260                 265                 270
   Leu Val Glu Thr Ser Asp Ala Leu Leu Cys Ile Ala Pro Val Phe Asn
           275                 280                 285
   Asp Tyr Ser Thr Val Gly Trp Ser Ala Trp Pro Lys Gly Pro Asn Val
       290                 295                 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Gly Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Leu Gly
   530 535 540
   Arg Lys Val Ala Ser Thr Asn Ala Arg Lys Thr Thr Leu Ala Leu Glu
   545 550 555 560
<210> 14
   <211> 560
   <212> PRT
   <213> artificial
<220>
   <223> künstlich hergestellt
<400> 14
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
                   245                 250                 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Gly Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Met Gly
   530 535 540
   Arg Lys Val Ala Ser Thr Asn Ala Arg Lys Thr Thr Leu Ala Leu Glu
   545 550 555 560
<210> 15
   <211> 560
   <212> PRT
   <213> artificial
<220>
   <223> künstlich hergestellt
<400> 15
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Gly Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Val Gly
   530 535 540
   Arg Lys Val Ala Ser Thr Asn Ala Arg Lys Thr Thr Leu Ala Leu Glu
   545 550 555 560
<210> 16
   <211> 560
   <212> PRT
   <213> artificial
<220>
   <223> künstlich hergestellt
<400> 16
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Gly Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Ala Gly
   530 535 540
   Arg Lys Val Ala Ser Thr Asn Ala Arg Lys Thr Thr Leu Ala Leu Glu
   545 550 555 560
<210> 17
   <211> 560
   <212> PRT
   <213> artificial
<220>
   <223> künstlich hergestellt
<400> 17
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
   85 90 95
   Ile Ser Gly Ala Pro Asn Ser Asn Asp Gln Gly Thr Gly His Ile Leu
   100 105 110
   His His Thr Ile Gly Lys Thr Asp Tyr Ser Tyr Gln Leu Glu Met Ala
   115 120 125
   Arg Gln Val Thr Cys Ala Ala Glu Ser Ile Thr Asp Ala His Ser Ala
   130 135 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
                   355                 360                 365
   His Ile Asn Ala Leu Leu Thr Ser Asn Thr Thr Leu Val Ala Glu Thr
       370                 375                 380
   Gly Asp Ser Trp Phe Asn Ala Met Arg Met Thr Leu Pro Arg Gly Ala
   385                 390                 395                 400
   Arg Val Glu Leu Glu Met Gln Trp Gly His Ile Gly Trp Ser Val Pro
                   405                 410                 415
   Ser Ala Phe Gly Asn Ala Met Gly Ser Gln Asp Arg Gln His Val Val
               420                 425                 430
   Met Val Gly Asp Gly Ser Phe Gln Leu Thr Ala Gln Glu Val Ala Gln
           435                 440                 445
   Met Val Arg Tyr Glu Leu Pro Val Ile Ile Phe Leu Ile Asn Asn Arg
       450                 455                 460
   Gly Tyr Val Gly Gly Ile Ala Ile His Asp Gly Pro Tyr Asn Tyr Ile
   465                 470                 475                 480
   Lys Asn Trp Asp Tyr Ala Gly Leu Met Glu Val Phe Asn Ala Gly Glu
                   485                 490                 495
   Gly His Gly Leu Gly Leu Lys Ala Thr Thr Pro Lys Glu Leu Thr Glu
               500                 505                 510
   Ala Ile Ala Arg Ala Lys Ala Asn Thr Arg Gly Pro Thr Leu Ile Glu
           515                 520                 525
   Cys Gln Ile Asp Arg Thr Asp Cys Thr Asp Met Leu Val Gln Tyr Gly
       530                 535                 540
   Arg Lys Val Ala Ser Thr Asn Ala Arg Lys Thr Thr Leu Ala Leu Glu
   545                 550                 555                 560
<210> 18
   <211> 560
   <212> PRT
   <213> artificial
<220>
   <223> künstlich hergestellt
<400> 18
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
   35 40 45
   Asn Glu Leu Asn Cys Gly Phe Ser Ala Glu Gly Tyr Ala Arg Ser Asn
   50 55 60
   Gly Ala Ala Ala Ala Val Val Thr Phe Ser Val Gly Ala Ile Ser Ala
   65 70 75 80
   Met Asn Ala Leu Gly Gly Ala Tyr Ala Glu Asn Leu Pro Val Ile Leu
   85 90 95
   Ile Ser Gly Ala Pro Asn Ser Asn Asp Gln Gly Thr Gly His Ile Leu
   100 105 110
   His His Thr Ile Gly Lys Thr Asp Tyr Ser Tyr Gln Leu Glu Met Ala
   115 120 125
   Arg Gln Val Thr Cys Ala Ala Glu Ser Ile Thr Asp Ala His Ser Ala
   130 135 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305                 310                 315                 320
   Gly Phe Thr Leu Arg Ala Phe Leu Gln Ala Leu Ala Glu Lys Ala Pro
                   325                 330                 335
   Ala Arg Pro Ala Ser Ala Gln Lys Ser Ser Val Pro Thr Cys Ser Leu
               340                 345                 350
   Thr Ala Thr Ser Asp Glu Ala Gly Leu Thr Asn Asp Glu Ile Val Arg
           355                 360                 365
   His Ile Asn Ala Leu Leu Thr Ser Asn Thr Thr Leu Val Ala Glu Thr
       370                 375                 380
   Gly Asp Ser Trp Phe Asn Ala Met Arg Met Thr Leu Pro Arg Gly Ala
   385                 390                 395                 400
   Arg Val Glu Leu Glu Met Gln Trp Gly His Ile Gly Trp Ser Val Pro
                   405                 410                 415
   Ser Ala Phe Gly Asn Ala Met Gly Ser Gln Asp Arg Gln His Val Val
               420                 425                 430
   Met Val Gly Asp Gly Ser Phe Gln Leu Thr Ala Gln Glu Val Ala Gln
           435                 440                 445
   Met Val Arg Tyr Glu Leu Pro Val Ile Ile Phe Leu Ile Asn Asn Arg
       450                 455                 460
   Gly Tyr Val Gly Gly Ile Ala Ile His Asp Gly Pro Tyr Asn Tyr Ile
   465                 470                 475                 480
   Lys Asn Trp Asp Tyr Ala Gly Leu Met Glu Val Phe Asn Ala Gly Glu
                   485                 490                 495
   Gly His Gly Leu Gly Leu Lys Ala Thr Thr Pro Lys Glu Leu Thr Glu
               500                 505                 510
   Ala Ile Ala Arg Ala Lys Ala Asn Thr Arg Gly Pro Thr Leu Ile Glu
           515                 520                 525
   Cys Gln Ile Asp Arg Thr Asp Cys Thr Asp Met Leu Val Gln Thr Gly
       530                 535                 540
   Arg Lys Val Ala Ser Thr Asn Ala Arg Lys Thr Thr Leu Ala Leu Glu
   545                 550                 555                 560
<210> 19
   <211> 560
   <212> PRT
   <213> artificial
<220>
   <223> künstlich hergestellt
<400> 19
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Gly Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Gly Gly
   530 535 540
   Arg Lys Val Ala Ser Thr Asn Ala Arg Lys Thr Thr Leu Ala Leu Glu
   545 550 555 560
<210> 20
   <211> 560
   <212> PRT
   <213> artificial
<220>
   <223> künstlich hergestellt
<400> 20
   Met Thr Tyr Thr Val Gly Met Tyr Leu Ala Glu Arg Leu Val Gln Ile
   1 5 10 15
   Gly Leu Lys His His Phe Ala Val Ala Gly Asp Tyr Asn Leu Val Leu
   20 25 30
   Leu Asp Gln Leu Leu Leu Asn Lys Asp Met Lys Gln Ile Tyr Cys Cys
   35 40 45
   Asn Glu Leu Asn Cys Gly Phe Ser Ala Glu Gly Tyr Ala Arg Ser Asn
   50 55 60
   Gly Ala Ala Ala Ala Val Val Thr Phe Ser Val Gly Ala Ile Ser Ala
   65 70 75 80
   Met Asn Ala Leu Gly Gly Ala Tyr Ala Glu Asn Leu Pro Val Ile Leu
   85 90 95
   Ile Ser Gly Ala Pro Asn Ser Asn Asp Gln Gly Thr Gly His Ile Leu
   100 105 110
   His His Thr Ile Gly Lys Thr Asp Tyr Ser Tyr Gln Leu Glu Met Ala
   115 120 125
   Arg Gln Val Thr Cys Ala Ala Glu Ser Ile Thr Asp Ala His Ser Ala
   130 135 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
                   245                 250                 255
   Phe Arg Gly Leu Tyr Trp Gly Glu Val Ser Asn Pro Gly Val Gln Glu
               260                 265                 270
   Leu Val Glu Thr Ser Asp Ala Leu Leu Cys Ile Ala Pro Val Phe Asn
           275                 280                 285
   Asp Tyr Ser Thr Val Gly Trp Ser Ala Trp Pro Lys Gly Pro Asn Val
       290                 295                 300
   Ile Leu Ala Glu Pro Asp Arg Val Thr Val Asp Gly Arg Ala Tyr Asp
   305                 310                 315                 320
   Gly Phe Thr Leu Arg Ala Phe Leu Gln Ala Leu Ala Glu Lys Ala Pro
                   325                 330                 335
   Ala Arg Pro Ala Ser Ala Gln Lys Ser Ser Val Pro Thr Cys Ser Leu
               340                 345                 350
   Thr Ala Thr Ser Asp Glu Ala Gly Leu Thr Asn Asp Glu Ile Val Arg
           355                 360                 365
   His Ile Asn Ala Leu Leu Thr Ser Asn Thr Thr Leu Val Ala Glu Thr
       370                 375                 380
   Gly Asp Ser Trp Phe Asn Ala Met Arg Met Thr Leu Pro Arg Gly Ala
   385                 390                 395                 400
   Arg Val Glu Leu Glu Met Gln Trp Gly His Ile Gly Trp Ser Val Pro
                   405                 410                 415
   Ser Ala Phe Gly Asn Ala Met Gly Ser Gln Asp Arg Gln His Val Val
               420                 425                 430
   Met Val Gly Asp Gly Ser Phe Gln Leu Thr Ala Gln Glu Val Ala Gln
           435                 440                 445
   Met Val Arg Tyr Glu Leu Pro Val Ile Ile Phe Leu Ile Asn Asn Arg
       450                 455                 460
   Gly Tyr Val Gly Gly Ile Ala Ile His Asp Gly Pro Tyr Asn Tyr Ile
   465                 470                     475                 480
   Lys Asn Trp Asp Tyr Ala Gly Leu Met Glu Val Phe Asn Ala Gly Glu
                   485                 490                 495
   Gly His Gly Leu Gly Leu Lys Ala Thr Thr Pro Lys Glu Leu Thr Glu
               500                 505                 510
   Ala Ile Ala Arg Ala Lys Ala Asn Thr Arg Gly Pro Thr Leu Ile Glu
           515                 520                 525
   Cys Gln Ile Asp Arg Thr Asp Cys Thr Asp Met Leu Val Gln Ser Gly
       530                 535                 540
   Arg Lys Val Ala Ser Thr Asn Ala Arg Lys Thr Thr Leu Ala Leu Glu
   545                 550                 555                 560
<210> 21
   <211> 560
   <212> PRT
   <213> artificial
<220>
   <223> künstlich hergestellt
<400> 21
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
   130 135 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Gly Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Cys Gly
   530 535 540
   Arg Lys Val Ala Ser Thr Asn Ala Arg Lys Thr Thr Leu Ala Leu Glu
   545 550 555 560
<210> 22
   <211> 560
   <212> PRT
   <213> artificial
<220>
   <223> künstlich hergestellt
<400> 22
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
   85 90 95
   Ile Ser Gly Ala Pro Asn Ser Asn Asp Gln Gly Thr Gly His Ile Leu
   100 105 110
   His His Thr Ile Gly Lys Thr Asp Tyr Ser Tyr Gln Leu Glu Met Ala
   115 120 125
   Arg Gln Val Thr Cys Ala Ala Glu Ser Ile Thr Asp Ala His Ser Ala
   130 135 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Ala Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln His Gly
   530 535 540
   Arg Lys Val Ala Ser Thr Asn Ala Arg Lys Thr Thr Leu Ala Leu Glu
   545 550 555 560
<210> 23
   <211> 560
   <212> PRT
   <213> artificial
<220>
   <223> künstlich hergestellt
<400> 23
   Met Thr Tyr Thr Val Gly Met Tyr Leu Ala Glu Arg Leu Val Gln Ile
   1 5 10 15
   Gly Leu Lys His His Phe Ala Val Ala Gly Asp Tyr Asn Leu Val Leu
   20 25 30
   Leu Asp Gln Leu Leu Leu Asn Lys Asp Met Lys Gln Ile Tyr Cys Cys
   35 40 45
   Asn Glu Leu Asn Cys Gly Phe Ser Ala Glu Gly Tyr Ala Arg Ser Asn
   50 55 60
   Gly Ala Ala Ala Ala Val Val Thr Phe Ser Val Gly Ala Ile Ser Ala
   65 70 75 80
   Met Asn Ala Leu Gly Gly Ala Tyr Ala Glu Asn Leu Pro Val Ile Leu
   85 90 95
   Ile Ser Gly Ala Pro Asn Ser Asn Asp Gln Gly Thr Gly His Ile Leu
   100 105 110
   His His Thr Ile Gly Lys Thr Asp Tyr Ser Tyr Gln Leu Glu Met Ala
   115 120 125
   Arg Gln Val Thr Cys Ala Ala Glu Ser Ile Thr Asp Ala His Ser Ala
   130 135 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Ala Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Phe Gly
   530 535 540
   Arg Lys Val Ala Ser Thr Asn Ala Arg Lys Thr Thr Leu Ala Leu Glu
   545 550 555 560
<210> 24
   <211> 560
   <212> PRT
   <213> artificial
<220>
   <223> künstlich hergestellt
<400> 24
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
                   245                 250                 255
   Phe Arg Gly Leu Tyr Trp Gly Glu Val Ser Asn Pro Gly Val Gln Glu
               260                 265                 270
   Leu Val Glu Thr Ser Asp Ala Leu Leu Cys Ile Ala Pro Val Phe Asn
           275                 280                 285
   Asp Tyr Ser Thr Val Gly Trp Ser Ala Trp Pro Lys Gly Pro Asn Val
       290                 295                 300
   Ile Leu Ala Glu Pro Asp Arg Val Thr Val Asp Gly Arg Ala Tyr Asp
   305                 310                 315                 320
   Gly Phe Thr Leu Arg Ala Phe Leu Gln Ala Leu Ala Glu Lys Ala Pro
                   325                 330                 335
   Ala Arg Pro Ala Ser Ala Gln Lys Ser Ser Val Pro Thr Cys Ser Leu
               340                 345                 350
   Thr Ala Thr Ser Asp Glu Ala Gly Leu Thr Asn Asp Glu Ile Val Arg
           355                 360                 365
   His Ile Asn Ala Leu Leu Thr Ser Asn Thr Thr Leu Val Ala Glu Thr
       370                 375                 380
   Gly Asp Ser Trp Phe Asn Ala Met Arg Met Thr Leu Pro Arg Gly Ala
   385                 390                 395                 400
   Arg Val Glu Leu Glu Met Gln Trp Gly His Ile Gly Trp Ser Val Pro
                   405                 410                 415
   Ser Ala Phe Gly Asn Ala Met Gly Ser Gln Asp Arg Gln His Val Val
               420                 425                 430
   Met Val Gly Asp Gly Ser Phe Gln Leu Thr Ala Gln Glu Val Ala Gln
           435                 440                 445
   Met Val Arg Tyr Glu Leu Pro Val Ile Ile Phe Leu Ile Asn Asn Arg
       450                 455                 460
   Gly Tyr Val Ala Gly Ile Ala Ile His Asp Gly Pro Tyr Asn Tyr Ile
   465                 470                 475                 480
   Lys Asn Trp Asp Tyr Ala Gly Leu Met Glu Val Phe Asn Ala Gly Glu
                   485                 490                 495
   Gly His Gly Leu Gly Leu Lys Ala Thr Thr Pro Lys Glu Leu Thr Glu
               500                 505                 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Pro Gly
   530 535 540
   Arg Lys Val Ala Ser Thr Asn Ala Arg Lys Thr Thr Leu Ala Leu Glu
   545 550 555 560
<210> 25
   <211> 560
   <212> PRT
   <213> artificial
<220>
   <223> künstlich hergestellt
<400> 25
   Met Thr Tyr Thr Val Gly Met Tyr Leu Ala Glu Arg Leu Val Gln Ile
   1 5 10 15
   Gly Leu Lys His His Phe Ala Val Ala Gly Asp Tyr Asn Leu Val Leu
   20 25 30
   Leu Asp Gln Leu Leu Leu Asn Lys Asp Met Lys Gln Ile Tyr Cys Cys
   35 40 45
   Asn Glu Leu Asn Cys Gly Phe Ser Ala Glu Gly Tyr Ala Arg Ser Asn
   50 55 60
   Gly Ala Ala Ala Ala Val Val Thr Phe Ser Val Gly Ala Ile Ser Ala
   65 70 75 80
   Met Asn Ala Leu Gly Gly Ala Tyr Ala Glu Asn Leu Pro Val Ile Leu
   85 90 95
   Ile Ser Gly Ala Pro Asn Ser Asn Asp Gln Gly Thr Gly His Ile Leu
   100 105 110
   His His Thr Ile Gly Lys Thr Asp Tyr Ser Tyr Gln Leu Glu Met Ala
   115 120 125
   Arg Gln Val Thr Cys Ala Ala Glu Ser Ile Thr Asp Ala His Ser Ala
   130 135 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Ala Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Ile Gly
   530 535 540
   Arg Lys Val Ala Ser Thr Asn Ala Arg Lys Thr Thr Leu Ala Leu Glu
   545 550 555 560
<210> 26
   <211> 560
   <212> PRT
   <213> artificial
<220>
   <223> künstlich hergestellt
<400> 26
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
                   245                 250                 255
   Phe Arg Gly Leu Tyr Trp Gly Glu Val Ser Asn Pro Gly Val Gln Glu
               260                 265                 270
   Leu Val Glu Thr Ser Asp Ala Leu Leu Cys Ile Ala Pro Val Phe Asn
           275                 280                 285
   Asp Tyr Ser Thr Val Gly Trp Ser Ala Trp Pro Lys Gly Pro Asn Val
       290                 295                 300
   Ile Leu Ala Glu Pro Asp Arg Val Thr Val Asp Gly Arg Ala Tyr Asp
   305                 310                 315                 320
   Gly Phe Thr Leu Arg Ala Phe Leu Gln Ala Leu Ala Glu Lys Ala Pro
                   325                 330                 335
   Ala Arg Pro Ala Ser Ala Gln Lys Ser Ser Val Pro Thr Cys Ser Leu
               340                 345                 350
   Thr Ala Thr Ser Asp Glu Ala Gly Leu Thr Asn Asp Glu Ile Val Arg
           355                 360                 365
   His Ile Asn Ala Leu Leu Thr Ser Asn Thr Thr Leu Val Ala Glu Thr
       370                 375                 380
   Gly Asp Ser Trp Phe Asn Ala Met Arg Met Thr Leu Pro Arg Gly Ala
   385                 390                 395                 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Ala Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Leu Gly
   530 535 540
   Arg Lys Val Ala Ser Thr Asn Ala Arg Lys Thr Thr Leu Ala Leu Glu
   545 550 555 560
<210> 27
   <211> 560
   <212> PRT
   <213> artificial
<220>
   <223> künstlich hergestellt
<400> 27
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65 70 75 80
   Met Asn Ala Leu Gly Gly Ala Tyr Ala Glu Asn Leu Pro Val Ile Leu
   85 90 95
   Ile Ser Gly Ala Pro Asn Ser Asn Asp Gln Gly Thr Gly His Ile Leu
   100 105 110
   His His Thr Ile Gly Lys Thr Asp Tyr Ser Tyr Gln Leu Glu Met Ala
   115 120 125
   Arg Gln Val Thr Cys Ala Ala Glu Ser Ile Thr Asp Ala His Ser Ala
   130 135 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
                   340                 345                 350
   Thr Ala Thr Ser Asp Glu Ala Gly Leu Thr Asn Asp Glu Ile Val Arg
           355                 360                 365
   His Ile Asn Ala Leu Leu Thr Ser Asn Thr Thr Leu Val Ala Glu Thr
       370                 375                 380
   Gly Asp Ser Trp Phe Asn Ala Met Arg Met Thr Leu Pro Arg Gly Ala
   385                 390                 395                 400
   Arg Val Glu Leu Glu Met Gln Trp Gly His Ile Gly Trp Ser Val Pro
                   405                 410                 415
   Ser Ala Phe Gly Asn Ala Met Gly Ser Gln Asp Arg Gln His Val Val
               420                 425                 430
   Met Val Gly Asp Gly Ser Phe Gln Leu Thr Ala Gln Glu Val Ala Gln
           435                 440                 445
   Met Val Arg Tyr Glu Leu Pro Val Ile Ile Phe Leu Ile Asn Asn Arg
       450                 455                 460
   Gly Tyr Val Ala Gly Ile Ala Ile His Asp Gly Pro Tyr Asn Tyr Ile
   465                 470                 475                 480
   Lys Asn Trp Asp Tyr Ala Gly Leu Met Glu Val Phe Asn Ala Gly Glu
                   485                 490                 495
   Gly His Gly Leu Gly Leu Lys Ala Thr Thr Pro Lys Glu Leu Thr Glu
               500                 505                 510
   Ala Ile Ala Arg Ala Lys Ala Asn Thr Arg Gly Pro Thr Leu Ile Glu
           515                 520                 525
   Cys Gln Ile Asp Arg Thr Asp Cys Thr Asp Met Leu Val Gln Met Gly
       530                 535                 540
   Arg Lys Val Ala Ser Thr Asn Ala Arg Lys Thr Thr Leu Ala Leu Glu
   545                 550                 555                 560
<210> 28
   <211> 560
   <212> PRT
   <213> artificial
<220>
   <223> künstlich hergestellt
<400> 28
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
   20 25 30
   Leu Asp Gln Leu Leu Leu Asn Lys Asp Met Lys Gln Ile Tyr Cys Cys
   35 40 45
   Asn Glu Leu Asn Cys Gly Phe Ser Ala Glu Gly Tyr Ala Arg Ser Asn
   50 55 60
   Gly Ala Ala Ala Ala Val Val Thr Phe Ser Val Gly Ala Ile Ser Ala
   65 70 75 80
   Met Asn Ala Leu Gly Gly Ala Tyr Ala Glu Asn Leu Pro Val Ile Leu
   85 90 95
   Ile Ser Gly Ala Pro Asn Ser Asn Asp Gln Gly Thr Gly His Ile Leu
   100 105 110
   His His Thr Ile Gly Lys Thr Asp Tyr Ser Tyr Gln Leu Glu Met Ala
   115 120 125
   Arg Gln Val Thr Cys Ala Ala Glu Ser Ile Thr Asp Ala His Ser Ala
   130 135 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Ala Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Val Gly
   530 535 540
   Arg Lys Val Ala Ser Thr Asn Ala Arg Lys Thr Thr Leu Ala Leu Glu
   545 550 555 560
<210> 29
   <211> 560
   <212> PRT
   <213> artificial
<220>
   <223> künstlich hergestellt
<400> 29
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Ala Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Ala Gly
   530 535 540
   Arg Lys Val Ala Ser Thr Asn Ala Arg Lys Thr Thr Leu Ala Leu Glu
   545 550 555 560
<210> 30
   <211> 560
   <212> PRT
   <213> artificial
<220>
   <223> künstlich hergestellt
<400> 30
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
                   245                 250                 255
   Phe Arg Gly Leu Tyr Trp Gly Glu Val Ser Asn Pro Gly Val Gln Glu
               260                 265                 270
   Leu Val Glu Thr Ser Asp Ala Leu Leu Cys Ile Ala Pro Val Phe Asn
           275                 280                 285
   Asp Tyr Ser Thr Val Gly Trp Ser Ala Trp Pro Lys Gly Pro Asn Val
       290                 295                 300
   Ile Leu Ala Glu Pro Asp Arg Val Thr Val Asp Gly Arg Ala Tyr Asp
   305                 310                 315                 320
   Gly Phe Thr Leu Arg Ala Phe Leu Gln Ala Leu Ala Glu Lys Ala Pro
                   325                 330                 335
   Ala Arg Pro Ala Ser Ala Gln Lys Ser Ser Val Pro Thr Cys Ser Leu
               340                 345                 350
   Thr Ala Thr Ser Asp Glu Ala Gly Leu Thr Asn Asp Glu Ile Val Arg
           355                 360                 365
   His Ile Asn Ala Leu Leu Thr Ser Asn Thr Thr Leu Val Ala Glu Thr
       370                 375                 380
   Gly Asp Ser Trp Phe Asn Ala Met Arg Met Thr Leu Pro Arg Gly Ala
   385                 390                 395                 400
   Arg Val Glu Leu Glu Met Gln Trp Gly His Ile Gly Trp Ser Val Pro
                   405                 410                 415
   Ser Ala Phe Gly Asn Ala Met Gly Ser Gln Asp Arg Gln His Val Val
               420                 425                 430
   Met Val Gly Asp Gly Ser Phe Gln Leu Thr Ala Gln Glu Val Ala Gln
           435                 440                 445
   Met Val Arg Tyr Glu Leu Pro Val Ile Ile Phe Leu Ile Asn Asn Arg
       450                 455                 460
   Gly Tyr Val Ala Gly Ile Ala Ile His Asp Gly Pro Tyr Asn Tyr Ile
   465                 470                 475                 480
   Lys Asn Trp Asp Tyr Ala Gly Leu Met Glu Val Phe Asn Ala Gly Glu
                   485                 490                 495
   Gly His Gly Leu Gly Leu Lys Ala Thr Thr Pro Lys Glu Leu Thr Glu
               500                 505                 510
   Ala Ile Ala Arg Ala Lys Ala Asn Thr Arg Gly Pro Thr Leu Ile Glu
           515                 520                 525
   Cys Gln Ile Asp Arg Thr Asp Cys Thr Asp Met Leu Val Gln Tyr Gly
       530                 535                 540
   Arg Lys Val Ala Ser Thr Asn Ala Arg Lys Thr Thr Leu Ala Leu Glu
   545                 550                 555                 560
<210> 31
   <211> 560
   <212> PRT
   <213> artificial
<220>
   <223> künstlich hergestellt
<400> 31
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
   115 120 125
   Arg Gln Val Thr Cys Ala Ala Glu Ser Ile Thr Asp Ala His Ser Ala
   130 135 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Ala Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Thr Gly
   530 535 540
   Arg Lys Val Ala Ser Thr Asn Ala Arg Lys Thr Thr Leu Ala Leu Glu
   545 550 555 560
<210> 32
   <211> 560
   <212> PRT
   <213> artificial
<220>
   <223> künstlich hergestellt
<400> 32
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65 70 75 80
   Met Asn Ala Leu Gly Gly Ala Tyr Ala Glu Asn Leu Pro Val Ile Leu
   85 90 95
   Ile Ser Gly Ala Pro Asn Ser Asn Asp Gln Gly Thr Gly His Ile Leu
   100 105 110
   His His Thr Ile Gly Lys Thr Asp Tyr Ser Tyr Gln Leu Glu Met Ala
   115 120 125
   Arg Gln Val Thr Cys Ala Ala Glu Ser Ile Thr Asp Ala His Ser Ala
   130 135 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Ala Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Gly Gly
   530 535 540
   Arg Lys Val Ala Ser Thr Asn Ala Arg Lys Thr Thr Leu Ala Leu Glu
   545 550 555 560
<210> 33
   <211> 560
   <212> PRT
   <213> artificial
<220>
   <223> künstlich hergestellt
<400> 33
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
                   245                 250                 255
   Phe Arg Gly Leu Tyr Trp Gly Glu Val Ser Asn Pro Gly Val Gln Glu
               260                 265                 270
   Leu Val Glu Thr Ser Asp Ala Leu Leu Cys Ile Ala Pro Val Phe Asn
                   275                     280                     285
   Asp Tyr Ser Thr Val Gly Trp Ser Ala Trp Pro Lys Gly Pro Asn Val
       290                 295                 300
   Ile Leu Ala Glu Pro Asp Arg Val Thr Val Asp Gly Arg Ala Tyr Asp
   305                 310                 315                 320
   Gly Phe Thr Leu Arg Ala Phe Leu Gln Ala Leu Ala Glu Lys Ala Pro
                   325                 330                 335
   Ala Arg Pro Ala Ser Ala Gln Lys Ser Ser Val Pro Thr Cys Ser Leu
               340                 345                 350
   Thr Ala Thr Ser Asp Glu Ala Gly Leu Thr Asn Asp Glu Ile Val Arg
           355                 360                 365
   His Ile Asn Ala Leu Leu Thr Ser Asn Thr Thr Leu Val Ala Glu Thr
       370                 375                 380
   Gly Asp Ser Trp Phe Asn Ala Met Arg Met Thr Leu Pro Arg Gly Ala
   385                 390                 395                 400
   Arg Val Glu Leu Glu Met Gln Trp Gly His Ile Gly Trp Ser Val Pro
                   405                 410                 415
   Ser Ala Phe Gly Asn Ala Met Gly Ser Gln Asp Arg Gln His Val Val
               420                 425                 430
   Met Val Gly Asp Gly Ser Phe Gln Leu Thr Ala Gln Glu Val Ala Gln
           435                 440                 445
   Met Val Arg Tyr Glu Leu Pro Val Ile Ile Phe Leu Ile Asn Asn Arg
       450                 455                 460
   Gly Tyr Val Ala Gly Ile Ala Ile His Asp Gly Pro Tyr Asn Tyr Ile
   465                 470                 475                 480
   Lys Asn Trp Asp Tyr Ala Gly Leu Met Glu Val Phe Asn Ala Gly Glu
                   485                 490                 495
   Gly His Gly Leu Gly Leu Lys Ala Thr Thr Pro Lys Glu Leu Thr Glu
               500                 505                 510
   Ala Ile Ala Arg Ala Lys Ala Asn Thr Arg Gly Pro Thr Leu Ile Glu
           515                 520                 525
   Cys Gln Ile Asp Arg Thr Asp Cys Thr Asp Met Leu Val Gln Ser Gly
       530                 535                 540
   Arg Lys Val Ala Ser Thr Asn Ala Arg Lys Thr Thr Leu Ala Leu Glu
   545 550 555 560
<210> 34
   <211> 560
   <212> PRT
   <213> artificial
<220>
   <223> künstlich hergestellt
<400> 34
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Ala Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
               500                 505                 510
   Ala Ile Ala Arg Ala Lys Ala Asn Thr Arg Gly Pro Thr Leu Ile Glu
           515                 520                 525
   Cys Gln Ile Asp Arg Thr Asp Cys Thr Asp Met Leu Val Gln Cys Gly
       530                 535                 540
   Arg Lys Val Ala Ser Thr Asn Ala Arg Lys Thr Thr Leu Ala Leu Glu
   545                 550                 555                 560
<210> 35
   <211> 560
   <212> PRT
   <213> artificial
<220>
   <223> künstlich hergestellt
<400> 35
   Met Thr Tyr Thr Val Gly Met Tyr Leu Ala Glu Arg Leu Val Gln Ile
   1 5 10 15
   Gly Leu Lys His His Phe Ala Val Ala Gly Asp Tyr Asn Leu Val Leu
   20 25 30
   Leu Asp Gln Leu Leu Leu Asn Lys Asp Met Lys Gln Ile Tyr Cys Cys
   35 40 45
   Asn Glu Leu Asn Cys Gly Phe Ser Ala Glu Gly Tyr Ala Arg Ser Asn
   50 55 60
   Gly Ala Ala Ala Ala Val Val Thr Phe Ser Val Gly Ala Ile Ser Ala
   65 70 75 80
   Met Asn Ala Leu Gly Gly Ala Tyr Ala Glu Asn Leu Pro Val Ile Leu
   85 90 95
   Ile Ser Gly Ala Pro Asn Ser Asn Asp Gln Gly Thr Gly His Ile Leu
   100 105 110
   His His Thr Ile Gly Lys Thr Asp Tyr Ser Tyr Gln Leu Glu Met Ala
   115 120 125
   Arg Gln Val Thr Cys Ala Ala Glu Ser Ile Thr Asp Ala His Ser Ala
   130 135 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Val Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln His Gly
   530 535 540
   Arg Lys Val Ala Ser Thr Asn Ala Arg Lys Thr Thr Leu Ala Leu Glu
   545 550 555 560
<210> 36
   <211> 560
   <212> PRT
   <213> artificial
<220>
   <223> künstlich hergestellt
<400> 36
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
                   245                 250                 255
   Phe Arg Gly Leu Tyr Trp Gly Glu Val Ser Asn Pro Gly Val Gln Glu
               260                 265                 270
   Leu Val Glu Thr Ser Asp Ala Leu Leu Cys Ile Ala Pro Val Phe Asn
           275                 280                 285
   Asp Tyr Ser Thr Val Gly Trp Ser Ala Trp Pro Lys Gly Pro Asn Val
       290                 295                 300
   Ile Leu Ala Glu Pro Asp Arg Val Thr Val Asp Gly Arg Ala Tyr Asp
   305                 310                 315                 320
   Gly Phe Thr Leu Arg Ala Phe Leu Gln Ala Leu Ala Glu Lys Ala Pro
                   325                 330                 335
   Ala Arg Pro Ala Ser Ala Gln Lys Ser Ser Val Pro Thr Cys Ser Leu
               340                 345                 350
   Thr Ala Thr Ser Asp Glu Ala Gly Leu Thr Asn Asp Glu Ile Val Arg
           355                 360                 365
   His Ile Asn Ala Leu Leu Thr Ser Asn Thr Thr Leu Val Ala Glu Thr
       370                 375                 380
   Gly Asp Ser Trp Phe Asn Ala Met Arg Met Thr Leu Pro Arg Gly Ala
   385                 390                 395                 400
   Arg Val Glu Leu Glu Met Gln Trp Gly His Ile Gly Trp Ser Val Pro
                   405                 410                 415
   Ser Ala Phe Gly Asn Ala Met Gly Ser Gln Asp Arg Gln His Val Val
               420                 425                 430
   Met Val Gly Asp Gly Ser Phe Gln Leu Thr Ala Gln Glu Val Ala Gln
           435                 440                 445
   Met Val Arg Tyr Glu Leu Pro Val Ile Ile Phe Leu Ile Asn Asn Arg
       450                 455                 460
   Gly Tyr Val Val Gly Ile Ala Ile His Asp Gly Pro Tyr Asn Tyr Ile
   465                 470                 475                 480
   Lys Asn Trp Asp Tyr Ala Gly Leu Met Glu Val Phe Asn Ala Gly Glu
                   485                 490                 495
   Gly His Gly Leu Gly Leu Lys Ala Thr Thr Pro Lys Glu Leu Thr Glu
               500                 505                 510
   Ala Ile Ala Arg Ala Lys Ala Asn Thr Arg Gly Pro Thr Leu Ile Glu
           515                 520                 525
   Cys Gln Ile Asp Arg Thr Asp Cys Thr Asp Met Leu Val Gln Phe Gly
       530                 535                 540
   Arg Lys Val Ala Ser Thr Asn Ala Arg Lys Thr Thr Leu Ala Leu Glu
   545                 550                 555                 560
<210> 37
   <211> 560
   <212> PRT
   <213> artificial
<220>
   <223> künstlich hergestellt
<400> 37
   Met Thr Tyr Thr Val Gly Met Tyr Leu Ala Glu Arg Leu Val Gln Ile
   1 5 10 15
   Gly Leu Lys His His Phe Ala Val Ala Gly Asp Tyr Asn Leu Val Leu
   20 25 30
   Leu Asp Gln Leu Leu Leu Asn Lys Asp Met Lys Gln Ile Tyr Cys Cys
   35 40 45
   Asn Glu Leu Asn Cys Gly Phe Ser Ala Glu Gly Tyr Ala Arg Ser Asn
   50 55 60
   Gly Ala Ala Ala Ala Val Val Thr Phe Ser Val Gly Ala Ile Ser Ala
   65 70 75 80
   Met Asn Ala Leu Gly Gly Ala Tyr Ala Glu Asn Leu Pro Val Ile Leu
   85 90 95
   Ile Ser Gly Ala Pro Asn Ser Asn Asp Gln Gly Thr Gly His Ile Leu
   100 105 110
   His His Thr Ile Gly Lys Thr Asp Tyr Ser Tyr Gln Leu Glu Met Ala
   115 120 125
   Arg Gln Val Thr Cys Ala Ala Glu Ser Ile Thr Asp Ala His Ser Ala
   130 135 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Val Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Pro Gly
   530 535 540
   Arg Lys Val Ala Ser Thr Asn Ala Arg Lys Thr Thr Leu Ala Leu Glu
   545 550 555 560
<210> 38
   <211> 560
   <212> PRT
   <213> artificial
<220>
   <223> künstlich hergestellt
<400> 38
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
                   245                 250                 255
   Phe Arg Gly Leu Tyr Trp Gly Glu Val Ser Asn Pro Gly Val Gln Glu
               260                 265                 270
   Leu Val Glu Thr Ser Asp Ala Leu Leu Cys Ile Ala Pro Val Phe Asn
           275                 280                 285
   Asp Tyr Ser Thr Val Gly Trp Ser Ala Trp Pro Lys Gly Pro Asn Val
       290                 295                 300
   Ile Leu Ala Glu Pro Asp Arg Val Thr Val Asp Gly Arg Ala Tyr Asp
   305                 310                 315                 320
   Gly Phe Thr Leu Arg Ala Phe Leu Gln Ala Leu Ala Glu Lys Ala Pro
                   325                 330                 335
   Ala Arg Pro Ala Ser Ala Gln Lys Ser Ser Val Pro Thr Cys Ser Leu
               340                 345                 350
   Thr Ala Thr Ser Asp Glu Ala Gly Leu Thr Asn Asp Glu Ile Val Arg
           355                 360                 365
   His Ile Asn Ala Leu Leu Thr Ser Asn Thr Thr Leu Val Ala Glu Thr
       370                 375                 380
   Gly Asp Ser Trp Phe Asn Ala Met Arg Met Thr Leu Pro Arg Gly Ala
   385                 390                 395                 400
   Arg Val Glu Leu Glu Met Gln Trp Gly His Ile Gly Trp Ser Val Pro
                   405                 410                 415
   Ser Ala Phe Gly Asn Ala Met Gly Ser Gln Asp Arg Gln His Val Val
               420                 425                 430
   Met Val Gly Asp Gly Ser Phe Gln Leu Thr Ala Gln Glu Val Ala Gln
           435                 440                 445
   Met Val Arg Tyr Glu Leu Pro Val Ile Ile Phe Leu Ile Asn Asn Arg
       450                 455                 460
   Gly Tyr Val Val Gly Ile Ala Ile His Asp Gly Pro Tyr Asn Tyr Ile
   465                 470                 475                 480
   Lys Asn Trp Asp Tyr Ala Gly Leu Met Glu Val Phe Asn Ala Gly Glu
                   485                 490                 495
   Gly His Gly Leu Gly Leu Lys Ala Thr Thr Pro Lys Glu Leu Thr Glu
               500                 505                 510
   Ala Ile Ala Arg Ala Lys Ala Asn Thr Arg Gly Pro Thr Leu Ile Glu
           515                 520                 525
   Cys Gln Ile Asp Arg Thr Asp Cys Thr Asp Met Leu Val Gln Ile Gly
       530                 535                 540
   Arg Lys Val Ala Ser Thr Asn Ala Arg Lys Thr Thr Leu Ala Leu Glu
   545                 550                 555                 560
<210> 39
   <211> 560
   <212> PRT
   <213> artificial
<220>
   <223> künstlich hergestellt
<400> 39
   Met Thr Tyr Thr Val Gly Met Tyr Leu Ala Glu Arg Leu Val Gln Ile
   1 5 10 15
   Gly Leu Lys His His Phe Ala Val Ala Gly Asp Tyr Asn Leu Val Leu
   20 25 30
   Leu Asp Gln Leu Leu Leu Asn Lys Asp Met Lys Gln Ile Tyr Cys Cys
   35 40 45
   Asn Glu Leu Asn Cys Gly Phe Ser Ala Glu Gly Tyr Ala Arg Ser Asn
   50 55 60
   Gly Ala Ala Ala Ala Val Val Thr Phe Ser Val Gly Ala Ile Ser Ala
   65 70 75 80
   Met Asn Ala Leu Gly Gly Ala Tyr Ala Glu Asn Leu Pro Val Ile Leu
   85 90 95
   Ile Ser Gly Ala Pro Asn Ser Asn Asp Gln Gly Thr Gly His Ile Leu
   100 105 110
   His His Thr Ile Gly Lys Thr Asp Tyr Ser Tyr Gln Leu Glu Met Ala
   115 120 125
   Arg Gln Val Thr Cys Ala Ala Glu Ser Ile Thr Asp Ala His Ser Ala
   130 135 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Val Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Leu Gly
   530 535 540
   Arg Lys Val Ala Ser Thr Asn Ala Arg Lys Thr Thr Leu Ala Leu Glu
   545 550 555 560
<210> 40
   <211> 560
   <212> PRT
   <213> artificial
<220>
   <223> künstlich hergestellt
<400> 40
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
                   245                 250                 255
   Phe Arg Gly Leu Tyr Trp Gly Glu Val Ser Asn Pro Gly Val Gln Glu
               260                 265                 270
   Leu Val Glu Thr Ser Asp Ala Leu Leu Cys Ile Ala Pro Val Phe Asn
           275                 280                 285
   Asp Tyr Ser Thr Val Gly Trp Ser Ala Trp Pro Lys Gly Pro Asn Val
       290                 295                 300
   Ile Leu Ala Glu Pro Asp Arg Val Thr Val Asp Gly Arg Ala Tyr Asp
   305                 310                 315                 320
   Gly Phe Thr Leu Arg Ala Phe Leu Gln Ala Leu Ala Glu Lys Ala Pro
                   325                 330                 335
   Ala Arg Pro Ala Ser Ala Gln Lys Ser Ser Val Pro Thr Cys Ser Leu
               340                 345                 350
   Thr Ala Thr Ser Asp Glu Ala Gly Leu Thr Asn Asp Glu Ile Val Arg
           355                 360                 365
   His Ile Asn Ala Leu Leu Thr Ser Asn Thr Thr Leu Val Ala Glu Thr
       370                 375                 380
   Gly Asp Ser Trp Phe Asn Ala Met Arg Met Thr Leu Pro Arg Gly Ala
   385                 390                 395                 400
   Arg Val Glu Leu Glu Met Gln Trp Gly His Ile Gly Trp Ser Val Pro
                   405                 410                 415
   Ser Ala Phe Gly Asn Ala Met Gly Ser Gln Asp Arg Gln His Val Val
               420                 425                 430
   Met Val Gly Asp Gly Ser Phe Gln Leu Thr Ala Gln Glu Val Ala Gln
           435                 440                 445
   Met Val Arg Tyr Glu Leu Pro Val Ile Ile Phe Leu Ile Asn Asn Arg
       450                 455                 460
   Gly Tyr Val Val Gly Ile Ala Ile His Asp Gly Pro Tyr Asn Tyr Ile
   465                 470                 475                 480
   Lys Asn Trp Asp Tyr Ala Gly Leu Met Glu Val Phe Asn Ala Gly Glu
                   485                 490                 495
   Gly His Gly Leu Gly Leu Lys Ala Thr Thr Pro Lys Glu Leu Thr Glu
               500                 505                 510
   Ala Ile Ala Arg Ala Lys Ala Asn Thr Arg Gly Pro Thr Leu Ile Glu
           515                 520                 525
   Cys Gln Ile Asp Arg Thr Asp Cys Thr Asp Met Leu Val Gln Met Gly
       530                 535                 540
   Arg Lys Val Ala Ser Thr Asn Ala Arg Lys Thr Thr Leu Ala Leu Glu
   545                 550                 555                 560
<210> 41
   <211> 560
   <212> PRT
   <213> artificial
<220>
   <223> künstlich hergestellt
<400> 41
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
   100 105 110
   His His Thr Ile Gly Lys Thr Asp Tyr Ser Tyr Gln Leu Glu Met Ala
   115 120 125
   Arg Gln Val Thr Cys Ala Ala Glu Ser Ile Thr Asp Ala His Ser Ala
   130 135 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Val Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Val Gly
   530 535 540
   Arg Lys Val Ala Ser Thr Asn Ala Arg Lys Thr Thr Leu Ala Leu Glu
   545 550 555 560
<210> 42
   <211> 560
   <212> PRT
   <213> artificial
<220>
   <223> künstlich hergestellt
<400> 42
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
                   245                 250                 255
   Phe Arg Gly Leu Tyr Trp Gly Glu Val Ser Asn Pro Gly Val Gln Glu
               260                 265                 270
   Leu Val Glu Thr Ser Asp Ala Leu Leu Cys Ile Ala Pro Val Phe Asn
           275                 280                 285
   Asp Tyr Ser Thr Val Gly Trp Ser Ala Trp Pro Lys Gly Pro Asn Val
       290                 295                 300
   Ile Leu Ala Glu Pro Asp Arg Val Thr Val Asp Gly Arg Ala Tyr Asp
   305                 310                 315                 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Val Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Ala Gly
   530 535 540
   Arg Lys Val Ala Ser Thr Asn Ala Arg Lys Thr Thr Leu Ala Leu Glu
   545 550 555 560
<210> 43
   <211> 560
   <212> PRT
   <213> artificial
<220>
   <223> künstlich hergestellt
<400> 43
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
                   245                 250                 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Val Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Tyr Gly
   530 535 540
   Arg Lys Val Ala Ser Thr Asn Ala Arg Lys Thr Thr Leu Ala Leu Glu
   545 550 555 560
<210> 44
   <211> 560
   <212> PRT
   <213> artificial
<220>
   <223> künstlich hergestellt
<400> 44
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Val Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
                   485                 490                 495
   Gly His Gly Leu Gly Leu Lys Ala Thr Thr Pro Lys Glu Leu Thr Glu
               500                 505                 510
   Ala Ile Ala Arg Ala Lys Ala Asn Thr Arg Gly Pro Thr Leu Ile Glu
           515                 520                 525
   Cys Gln Ile Asp Arg Thr Asp Cys Thr Asp Met Leu Val Gln Thr Gly
       530                 535                 540
   Arg Lys Val Ala Ser Thr Asn Ala Arg Lys Thr Thr Leu Ala Leu Glu
   545                 550                 555                 560
<210> 45
   <211> 560
   <212> PRT
   <213> artificial
<220>
   <223> künstlich hergestellt
<400> 45
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
                   420                 425                 430
   Met Val Gly Asp Gly Ser Phe Gln Leu Thr Ala Gln Glu Val Ala Gln
           435                 440                 445
   Met Val Arg Tyr Glu Leu Pro Val Ile Ile Phe Leu Ile Asn Asn Arg
       450                 455                 460
   Gly Tyr Val Val Gly Ile Ala Ile His Asp Gly Pro Tyr Asn Tyr Ile
   465                 470                 475                 480
   Lys Asn Trp Asp Tyr Ala Gly Leu Met Glu Val Phe Asn Ala Gly Glu
                   485                 490                 495
   Gly His Gly Leu Gly Leu Lys Ala Thr Thr Pro Lys Glu Leu Thr Glu
               500                 505                 510
   Ala Ile Ala Arg Ala Lys Ala Asn Thr Arg Gly Pro Thr Leu Ile Glu
           515                 520                 525
   Cys Gln Ile Asp Arg Thr Asp Cys Thr Asp Met Leu Val Gln Gly Gly
       530                 535                 540
   Arg Lys Val Ala Ser Thr Asn Ala Arg Lys Thr Thr Leu Ala Leu Glu
   545                 550                 555                 560
<210> 46
   <211> 560
   <212> PRT
   <213> artificial
<220>
   <223> künstlich hergestellt
<400> 46
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
   100 105 110
   His His Thr Ile Gly Lys Thr Asp Tyr Ser Tyr Gln Leu Glu Met Ala
   115 120 125
   Arg Gln Val Thr Cys Ala Ala Glu Ser Ile Thr Asp Ala His Ser Ala
   130 135 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Val Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Ser Gly
   530 535 540
   Arg Lys Val Ala Ser Thr Asn Ala Arg Lys Thr Thr Leu Ala Leu Glu
   545 550 555 560
<210> 47
   <211> 560
   <212> PRT
   <213> artificial
<220>
   <223> künstlich hergestellt
<400> 47
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
                   245                 250                 255
   Phe Arg Gly Leu Tyr Trp Gly Glu Val Ser Asn Pro Gly Val Gln Glu
               260                 265                 270
   Leu Val Glu Thr Ser Asp Ala Leu Leu Cys Ile Ala Pro Val Phe Asn
           275                 280                 285
   Asp Tyr Ser Thr Val Gly Trp Ser Ala Trp Pro Lys Gly Pro Asn Val
       290                 295                 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Val Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Cys Gly
   530 535 540
   Arg Lys Val Ala Ser Thr Asn Ala Arg Lys Thr Thr Leu Ala Leu Glu
   545 550 555 560
<210> 48
   <211> 1680
   <212> DNA
   <213> artificial
<220>
   <223> künstlich hergestellt
<400> 48
   atgacctata ctgttggcat gtatcttgca gaacgccttg tacagatcgg gctgaagcat      60
   cacttcgccg tggcgggcga ctacaatctc gttcttctgg atcagttgct cctcaacaag     120
   gacatgaaac agatctattg ctgcaatgag ttgaactgtg gcttcagcgc ggaaggctac     180
   gcccgttcta acggggctgc ggcagcggtt gtcaccttca gcgttggcgc catttccgcc     240
   atgaacgccc tcggcggcgc ctatgccgaa aacctgccgg ttatcctgat ttccggcgcg     300
   cccaacagca atgatcaggg cacaggtcat atcctgcatc acacaatcgg caagacggat     360
   tacagctacc agcttgaaat ggcccgtcag gtcacctgtg ccgccgaaag cattaccgac     420
   gctcactccg ccccggccaa gattgaccac gtcattcgca cggcgctgcg cgagcgtaag     480
   ccggcctatc tggacatcgc gtgcaacatt gcctccgagc cctgcgtgcg gcctggccct     540
   gtcagcagcc tgctgtccga gcctgaaatc gaccacacga gcctgaaggc cgcagtggac     600
   gccacggttg ccttgctgga aaaatcggcc agccccgtca tgctgctggg cagcaagctg     660
   cgggccgcca acgcactggc cgcaaccgaa acgctggcag acaagctgca atgcgcggtg     720
   accatcatgg cggccgcgaa aggctttttc cccgaagacc acgcgggttt ccgcggcctg     780
   tactggggcg aagtctcgaa ccccggcgtg caggaactgg tggagacctc cgacgcactg     840
   ctgtgcatcg cccccgtatt caacgactat tcaacagtcg gctggtcggc atggcccaag     900
   ggccccaatg tgattctggc tgagcccgac cgcgtaacgg tcgatggccg cgcctatgac     960
   ggctttaccc tgcgcgcctt cctgcaggct ctggcggaaa aagcccccgc gcgcccggcc    1020
   tccgcacaga aaagcagcgt cccgacgtgc tcgctcaccg cgacatccga tgaagccggt    1080
   ctgacgaatg acgaaatcgt ccgtcatatc aacgccctgc tgacatcaaa cacgacgctg    1140
   gtggcagaaa ccggcgattc atggttcaat gccatgcgca tgaccctgcc gcgcggtgcg    1200
   cgcgtggaac tggaaatgca gtggggccat atcggctggt ccgtgccctc cgccttcggc    1260
   aatgccatgg gctcgcagga ccgccagcat gtggtgatgg taggcgatgg ctccttccag    1320
   cttaccgcgc aggaagtggc tcagatggtg cgctacgaac tgcccgtcat tatctttctg    1380
   atcaacaacc gtggctatgt cgccggcatc gccattcatg acggcccgta caactatatc    1440
   aagaactggg attacgccgg cctgatggaa gtcttcaacg ccggagaagg ccatggactt    1500
   ggcctgaaag ccaccacccc gaaggaactg acagaagcca tcgccagggc aaaagccaat    1560
   acccgcggcc cgacgctgat cgaatgccag atcgaccgca cggactgcac ggatatgctg    1620
   gttcaatttg gccgcaaggt tgcctcaacc aacgcgcgca agaccactct ggccctcgag    1680
<210> 49
   <211> 7041
   <212> DNA
   <213> artificial
<220>
   <223> künstlich hergestellt
<400> 49
   tggcgaatgg gacgcgccct gtagcggcgc attaagcgcg gcgggtgtgg tggttacgcg 60
   cagcgtgacc gctacacttg ccagcgccct agcgcccgct cctttcgctt tcttcccttc 120
   ctttctcgcc acgttcgccg gctttccccg tcaagctcta aatcgggggc tccctttagg 180
   gttccgattt agtgctttac ggcacctcga ccccaaaaaa cttgattagg gtgatggttc 240
   acgtagtggg ccatcgccct gatagacggt ttttcgccct ttgacgttgg agtccacgtt 300
   ctttaatagt ggactcttgt tccaaactgg aacaacactc aaccctatct cggtctattc 360
   ttttgattta taagggattt tgccgatttc ggcctattgg ttaaaaaatg agctgattta 420
   acaaaaattt aacgcgaatt ttaacaaaat attaacgttt acaatttcag gtggcacttt 480
   tcggggaaat gtgcgcggaa cccctatttg tttatttttc taaatacatt caaatatgta 540
   tccgctcatg agacaataac cctgataaat gcttcaataa tattgaaaaa ggaagagtat 600
   gagtattcaa catttccgtg tcgcccttat tccctttttt gcggcatttt gccttcctgt 660
   ttttgctcac ccagaaacgc tggtgaaagt aaaagatgct gaagatcagt tgggtgcacg 720
   agtgggttac atcgaactgg atctcaacag cggtaagatc cttgagagtt ttcgccccga 780
   agaacgtttt ccaatgatga gcacttttaa agttctgcta tgtggcgcgg tattatcccg 840
   tattgacgcc gggcaagagc aactcggtcg ccgcatacac tattctcaga atgacttggt 900
   tgagtactca ccagtcacag aaaagcatct tacggatggc atgacagtaa gagaattatg 960
   cagtgctgcc ataaccatga gtgataacac tgcggccaac ttacttctga caacgatcgg 1020
   aggaccgaag gagctaaccg cttttttgca caacatgggg gatcatgtaa ctcgccttga 1080
   tcgttgggaa ccggagctga atgaagccat accaaacgac gagcgtgaca ccacgatgcc 1140
   tgcagcaatg gcaacaacgt tgcgcaaact attaactggc gaactactta ctctagcttc 1200
   ccggcaacaa ttaatagact ggatggaggc ggataaagtt gcaggaccac ttctgcgctc 1260
   ggcccttccg gctggctggt ttattgctga taaatctgga gccggtgagc gtgggtctcg 1320
   cggtatcatt gcagcactgg ggccagatgg taagccctcc cgtatcgtag ttatctacac 1380
   gacggggagt caggcaacta tggatgaacg aaatagacag atcgctgaga taggtgcctc 1440
   actgattaag cattggtaac tgtcagacca agtttactca tatatacttt agattgattt 1500
   aaaacttcat ttttaattta aaaggatcta ggtgaagatc ctttttgata atctcatgac 1560
   caaaatccct taacgtgagt tttcgttcca ctgagcgtca gaccccgtag aaaagatcaa 1620
   aggatcttct tgagatcctt tttttctgcg cgtaatctgc tgcttgcaaa caaaaaaacc 1680
   accgctacca gcggtggttt gtttgccgga tcaagagcta ccaactcttt ttccgaaggt 1740
   aactggcttc agcagagcgc agataccaaa tactgtcctt ctagtgtagc cgtagttagg 1800
   ccaccacttc aagaactctg tagcaccgcc tacatacctc gctctgctaa tcctgttacc 1860
   agtggctgct gccagtggcg ataagtcgtg tcttaccggg ttggactcaa gacgatagtt 1920
   accggataag gcgcagcggt cgggctgaac ggggggttcg tgcacacagc ccagcttgga 1980
   gcgaacgacc tacaccgaac tgagatacct acagcgtgag ctatgagaaa gcgccacgct 2040
   tcccgaaggg agaaaggcgg acaggtatcc ggtaagcggc agggtcggaa caggagagcg 2100
   cacgagggag cttccagggg gaaacgcctg gtatctttat agtcctgtcg ggtttcgcca 2160
   cctctgactt gagcgtcgat ttttgtgatg ctcgtcaggg gggcggagcc tatggaaaaa 2220
   cgccagcaac gcggcctttt tacggttcct ggccttttgc tggccttttg ctcacatgtt 2280
   ctttcctgcg ttatcccctg attctgtgga taaccgtatt accgcctttg agtgagctga 2340
   taccgctcgc cgcagccgaa cgaccgagcg cagcgagtca gtgagcgagg aagcggaaga 2400
   gcgcctgatg cggtattttc tccttacgca tctgtgcggt atttcacacc gcatatatgg 2460
   tgcactctca gtacaatctg ctctgatgcc gcatagttaa gccagtatac actccgctat 2520
   cgctacgtga ctgggtcatg gctgcgcccc gacacccgcc aacacccgct gacgcgccct 2580
   gacgggcttg tctgctcccg gcatccgctt acagacaagc tgtgaccgtc tccgggagct 2640
   gcatgtgtca gaggttttca ccgtcatcac cgaaacgcgc gaggcagctg cggtaaagct 2700
   catcagcgtg gtcgtgaagc gattcacaga tgtctgcctg ttcatccgcg tccagctcgt 2760
   tgagtttctc cagaagcgtt aatgtctggc ttctgataaa gcgggccatg ttaagggcgg 2820
   ttttttcctg tttggtcact gatgcctccg tgtaaggggg atttctgttc atgggggtaa 2880
   tgataccgat gaaacgagag aggatgctca cgatacgggt tactgatgat gaacatgccc 2940
   ggttactgga acgttgtgag ggtaaacaac tggcggtatg gatgcggcgg gaccagagaa 3000
   aaatcactca gggtcaatgc cagcgcttcg ttaatacaga tgtaggtgtt ccacagggta 3060
   gccagcagca tcctgcgatg cagatccgga acataatggt gcagggcgct gacttccgcg 3120
   tttccagact ttacgaaaca cggaaaccga agaccattca tgttgttgct caggtcgcag 3180
   acgttttgca gcagcagtcg cttcacgttc gctcgcgtat cggtgattca ttctgctaac 3240
   cagtaaggca accccgccag cctagccggg tcctcaacga caggagcacg atcatgcgca 3300
   cccgtggggc cgccatgccg gcgataatgg cctgcttctc gccgaaacgt ttggtggcgg 3360
   gaccagtgac gaaggcttga gcgagggcgt gcaagattcc gaataccgca agcgacaggc 3420
   cgatcatcgt cgcgctccag cgaaagcggt cctcgccgaa aatgacccag agcgctgccg 3480
   gcacctgtcc tacgagttgc atgataaaga agacagtcat aagtgcggcg acgatagtca 3540
   tgccccgcgc ccaccggaag gagctgactg ggttgaaggc tctcaagggc atcggtcgag 3600
   atcccggtgc ctaatgagtg agctaactta cattaattgc gttgcgctca ctgcccgctt 3660
   tccagtcggg aaacctgtcg tgccagctgc attaatgaat cggccaacgc gcggggagag 3720
   gcggtttgcg tattgggcgc cagggtggtt tttcttttca ccagtgagac gggcaacagc 3780
   tgattgccct tcaccgcctg gccctgagag agttgcagca agcggtccac gctggtttgc 3840
   cccagcaggc gaaaatcctg tttgatggtg gttaacggcg ggatataaca tgagctgtct 3900
   tcggtatcgt cgtatcccac taccgagata tccgcaccaa cgcgcagccc ggactcggta 3960
   atggcgcgca ttgcgcccag cgccatctga tcgttggcaa ccagcatcgc agtgggaacg 4020
   atgccctcat tcagcatttg catggtttgt tgaaaaccgg acatggcact ccagtcgcct 4080
   tcccgttccg ctatcggctg aatttgattg cgagtgagat atttatgcca gccagccaga 4140
   cgcagacgcg ccgagacaga acttaatggg cccgctaaca gcgcgatttg ctggtgaccc 4200
   aatgcgacca gatgctccac gcccagtcgc gtaccgtctt catgggagaa aataatactg 4260
   ttgatgggtg tctggtcaga gacatcaaga aataacgccg gaacattagt gcaggcagct 4320
   tccacagcaa tggcatcctg gtcatccagc ggatagttaa tgatcagccc actgacgcgt 4380
   tgcgcgagaa gattgtgcac cgccgcttta caggcttcga cgccgcttcg ttctaccatc 4440
   gacaccacca cgctggcacc cagttgatcg gcgcgagatt taatcgccgc gacaatttgc 4500
   gacggcgcgt gcagggccag actggaggtg gcaacgccaa tcagcaacga ctgtttgccc 4560
   gccagttgtt gtgccacgcg gttgggaatg taattcagct ccgccatcgc cgcttccact 4620
   ttttcccgcg ttttcgcaga aacgtggctg gcctggttca ccacgcggga aacggtctga 4680
   taagagacac cggcatactc tgcgacatcg tataacgtta ctggtttcac attcaccacc 4740
   ctgaattgac tctcttccgg gcgctatcat gccataccgc gaaaggtttt gcgccattcg 4800
   atggtgtccg ggatctcgac gctctccctt atgcgactcc tgcattagga agcagcccag 4860
   tagtaggttg aggccgttga gcaccgccgc cgcaaggaat ggtgcatgca aggagatggc 4920
   gcccaacagt cccccggcca cggggcctgc caccataccc acgccgaaac aagcgctcat 4980
   gagcccgaag tggcgagccc gatcttcccc atcggtgatg tcggcgatat aggcgccagc 5040
   aaccgcacct gtggcgccgg tgatgccggc cacgatgcgt ccggcgtaga ggatcgagat 5100
   ctcgatcccg cgaaattaat acgactcact ataggggaat tgtgagcgga taacaattcc 5160
   cctctagaaa taattttgtt taactttaag aaggagatat acatatgacc tatactgttg 5220
   gcatgtatct tgcagaacgc cttgtacaga tcgggctgaa gcatcacttc gccgtggcgg 5280
   gcgactacaa tctcgttctt ctggatcagt tgctcctcaa caaggacatg aaacagatct 5340
   attgctgcaa tgagttgaac tgtggcttca gcgcggaagg ctacgcccgt tctaacgggg 5400
   ctgcggcagc ggttgtcacc ttcagcgttg gcgccatttc cgccatgaac gccctcggcg 5460
   gcgcctatgc cgaaaacctg ccggttatcc tgatttccgg cgcgcccaac agcaatgatc 5520
   agggcacagg tcatatcctg catcacacaa tcggcaagac ggattacagc taccagcttg 5580
   aaatggcccg tcaggtcacc tgtgccgccg aaagcattac cgacgctcac tccgccccgg 5640
   ccaagattga ccacgtcatt cgcacggcgc tgcgcgagcg taagccggcc tatctggaca 5700
   tcgcgtgcaa cattgcctcc gagccctgcg tgcggcctgg ccctgtcagc agcctgctgt 5760
   ccgagcctga aatcgaccac acgagcctga aggccgcagt ggacgccacg gttgccttgc 5820
   tggaaaaatc ggccagcccc gtcatgctgc tgggcagcaa gctgcgggcc gccaacgcac 5880
   tggccgcaac cgaaacgctg gcagacaagc tgcaatgcgc ggtgaccatc atggcggccg 5940
   cgaaaggctt tttccccgaa gaccacgcgg gtttccgcgg cctgtactgg ggcgaagtct 6000
   cgaaccccgg cgtgcaggaa ctggtggaga cctccgacgc actgctgtgc atcgcccccg 6060
   tattcaacga ctattcaaca gtcggctggt cggcatggcc caagggcccc aatgtgattc 6120
   tggctgagcc cgaccgcgta acggtcgatg gccgcgccta tgacggcttt accctgcgcg 6180
   ccttcctgca ggctctggcg gaaaaagccc ccgcgcgccc ggcctccgca cagaaaagca 6240
   gcgtcccgac gtgctcgctc accgcgacat ccgatgaagc cggtctgacg aatgacgaaa 6300
   tcgtccgtca tatcaacgcc ctgctgacat caaacacgac gctggtggca gaaaccggcg 6360
   attcatggtt caatgccatg cgcatgaccc tgccgcgcgg tgcgcgcgtg gaactggaaa 6420
   tgcagtgggg ccatatcggc tggtccgtgc cctccgcctt cggcaatgcc atgggctcgc 6480
   aggaccgcca gcatgtggtg atggtaggcg atggctcctt ccagcttacc gcgcaggaag 6540
   tggctcagat ggtgcgctac gaactgcccg tcattatctt tctgatcaac aaccgtggct 6600
   atgtcgccgg catcgccatt catgacggcc cgtacaacta tatcaagaac tgggattacg 6660
   ccggcctgat ggaagtcttc aacgccggag aaggccatgg acttggcctg aaagccacca 6720
   ccccgaagga actgacagaa gccatcgcca gggcaaaagc caatacccgc ggcccgacgc 6780
   tgatcgaatg ccagatcgac cgcacggact gcacggatat gctggttcaa tttggccgca 6840
   aggttgcctc aaccaacgcg cgcaagacca ctctggccct cgagcaccac caccaccacc 6900
   actgagatcc ggctgctaac aaagcccgaa aggaagctga gttggctgct gccaccgctg 6960
   agcaataact agcataaccc cttggggcct ctaaacgggt cttgaggggt tttttgctga 7020
   aaggaggaac tatatccgga t 7041
<210> 50
   <211> 34
   <212> DNA
   <213> artificial
<220>
   <223> künstlich hergestellt
<400> 50
   accttgcggc cgaattgaac cagcatatcc gtgc 34
<210> 51
   <211> 34
   <212> DNA
   <213> artificial
<220>
   <223> künstlich hergestellt
<400> 51
   gcacggatat gctggttcaa ttcggccgca aggt 34
<210> 52
   <211> 1680
   <212> DNA
   <213> artificial
<220>
   <223> künstlich hergestellt
<400> 52
   atgacctata ctgttggcat gtatcttgca gaacgccttg tacagatcgg gctgaagcat      60
   cacttcgccg tggcgggcga ctacaatctc gttcttctgg atcagttgct cctcaacaag     120
   gacatgaaac agatctattg ctgcaatgag ttgaactgtg gcttcagcgc ggaaggctac     180
   gcccgttcta acggggctgc ggcagcggtt gtcaccttca gcgttggcgc catttccgcc     240
   atgaacgccc tcggcggcgc ctatgccgaa aacctgccgg ttatcctgat ttccggcgcg     300
   cccaacagca atgatcaggg cacaggtcat atcctgcatc acacaatcgg caagacggat     360
   tacagctacc agcttgaaat ggcccgtcag gtcacctgtg ccgccgaaag cattaccgac     420
   gctcactccg ccccggccaa gattgaccac gtcattcgca cggcgctgcg cgagcgtaag     480
   ccggcctatc tggacatcgc gtgcaacatt gcctccgagc cctgcgtgcg gcctggccct     540
   gtcagcagcc tgctgtccga gcctgaaatc gaccacacga gcctgaaggc cgcagtggac     600
   gccacggttg ccttgctgga aaaatcggcc agccccgtca tgctgctggg cagcaagctg     660
   cgggccgcca acgcactggc cgcaaccgaa acgctggcag acaagctgca atgcgcggtg     720
   accatcatgg cggccgcgaa aggctttttc cccgaagacc acgcgggttt ccgcggcctg     780
   tactggggcg aagtctcgaa ccccggcgtg caggaactgg tggagacctc cgacgcactg     840
   ctgtgcatcg cccccgtatt caacgactat tcaacagtcg gctggtcggc atggcccaag     900
   ggccccaatg tgattctggc tgagcccgac cgcgtaacgg tcgatggccg cgcctatgac     960
   ggctttaccc tgcgcgcctt cctgcaggct ctggcggaaa aagcccccgc gcgcccggcc    1020
   tccgcacaga aaagcagcgt cccgacgtgc tcgctcaccg cgacatccga tgaagccggt    1080
   ctgacgaatg acgaaatcgt ccgtcatatc aacgccctgc tgacatcaaa cacgacgctg    1140
   gtggcagaaa ccggcgattc atggttcaat gccatgcgca tgaccctgcc gcgcggtgcg    1200
   cgcgtggaac tggaaatgca gtggggccat atcggctggt ccgtgccctc cgccttcggc    1260
   aatgccatgg gctcgcagga ccgccagcat gtggtgatgg taggcgatgg ctccttccag    1320
   cttaccgcgc aggaagtggc tcagatggtg cgctacgaac tgcccgtcat tatctttctg    1380
   atcaacaacc gtggctatgt cgccggcatc gccattcatg acggcccgta caactatatc    1440
   aagaactggg attacgccgg cctgatggaa gtcttcaacg ccggagaagg ccatggactt    1500
   ggcctgaaag ccaccacccc gaaggaactg acagaagcca tcgccagggc aaaagccaat    1560
   acccgcggcc cgacgctgat cgaatgccag atcgaccgca cggactgcac ggatatgctg    1620
   gttcaatggg gccgcaaggt tgcctcaacc aacgcgcgca agaccactct ggccctcgag    1680
<210> 53
   <211> 560
   <212> PRT
   <213> artificial
<220>
   <223> künstlich hergestellt
<400> 53
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
                   245                 250                 255
   Phe Arg Gly Leu Tyr Trp Gly Glu Val Ser Asn Pro Gly Val Gln Glu
               260                 265                 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Ile Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Trp Gly
   530 535 540
   Arg Lys Val Ala Ser Thr Asn Ala Arg Lys Thr Thr Leu Ala Leu Glu
   545 550 555 560

## Beschreibung

Die Erfindung betrifft eine Lyase sowie ein Verfahren zur asymmetrischen Synthese von (*S*)-Phenylacetyl-carbinol.

(S)-Phenylacetylcarbinol ist ein wertvoller chiraler Baustein in organischen Synthesen und kann zur Synthese von Feinchemikalien und Pharmazeutika genutzt werden. Nach dem bisherigen Stand der Technik sind keine Methoden bekannt, bei den (*S*)-Phenylacetylcarbinol (S)-PAC mittels asymmetrischer Synthese aus unchiralen, kostengünstigen Verbindungen in optischen Reinheiten >89 % ee generiert werden kann. Hohe optische Reinheiten sind jedoch von entschiedener Bedeutung bei der Herstellung von Feinchemikalien, bzw. Pharmazeutika.

Nach dem Stand der Technik sind verschiedene Methoden bekannt um (*S*)-Phenylacetylcarbinol herzustellen.

Zum einen sind chemische Synthesen bekannt.
Die auf chemischer asymmetrischer Synthese beruhenden Methoden zur Herstellung von (S)-PAC, generieren einen ee von 68 % bzw. 86 %. Die Verfahren sind in den Veröffentlichungen von Davis, Franklin A.; Sheppard, Aurelia C.; Lal, G. Sankar Tetrahedron Letters, 1989, vol. 30, 7 p. 779 - 782 und Adam, Waldemar; Fell, Rainer T.; Stegmann, Veit R.; Saha-Moeller, Chantu R. Journal of the American Chemical Society, 1998, vol. 120, 4 p. 708 - 714 beschrieben. Zudem gibt es Methoden, bei denen (*S*)-PAC nur als Nebenprodukt entsteht und (R)-PAC im Enantiomerenüberschuss vorliegt wie z. B. bei folgenden Reaktionen, wie der Reduktion von 1-Phenylpropan-1,2-dion, die in den Veröffentlichungen von Toukoniitty, Esa; Maeki-Arvela, Paeivi; Kuzma, Marek; Villela, Alexandre; Kalantar Neyestanaki, Ahmad; Salmi, Tapio; Sjoeholm, Rainer; Leino, Reko; Laine, Ensio; Murzin, Dmitry Yu, Journal of Catalysis, 2001, vol. 204, 2 p. 281 - 291 und der Synthese ausgehend von Benzaldehyd die von Fleming, Steven A.; Carroll, Sean M.; Hirschi, Jennifer; Liu, Renmao; Pace, J. Lee; Redd, J. Ty Tetrahedron Letters, 2004, vol. 45, 17 p. 3341 - 3343 und der Reaktion von 2-Hydroxy-2-Phenylacetonitril von Brussee, J.; Roos, E. C.; Gen, A. Van Der Tetrahedron Letters, 1988, vol. 29, 35 p. 4485 - 4488 beschrieben sind.

Darüber hinaus ist eine Synthese beschrieben, bei der der chirale Baustein, 1-Phenylpropan-1,2-diol, zu (S)-PAC oxidiert werden kann. (*S*)-PAC entsteht mit einem Enantiomerenüberschuss von 91 %, wie von Zi-Qiang Rong, Hui-Jie Pan, Hai-Long Yan, und Yu Zhao Organic Letters, 2014, 16 (1), pp 208-211 beschrieben, bzw. 69 % wie von Waldemar Adam, Chantu R. Saha-Moeller, und Cong-Gui Zhao, Journal of Organic Chemistry, 64(20), 7492-7497; 1999 beschrieben wurde, ist aber zudem mit einem Regioisomer verunreinigt, das aufwändig abgetrennt werden muss.

Weiterhin ist eine enzymatische asymmetrische Synthese bekannt die in der Dissertation von Älvaro Gómez Baraibar mit dem Titel "Development of a biocatalytic production process for (S)-alpha-hydroxy ketones" aus dem Jahr 2013 beschrieben ist. Verwendet man dieses Enzym gemäß dieser Dissertation heterolog in *Escherichia coli* exprimert für die Synthese in ganzen Zellen, beträgt die optische Reinheit von (*S*)-PAC ∼43 % ee.

Diese einzige enzymatische, asymmetrische Synthese von (S)-PAC wurde in einer Carboligationsreaktion ausgehend von Benzaldehyd und Acetaldehyd, bzw. Benzaldehyd und Pyruvat beschrieben. Die Reaktion wird von einer Variante des Enzyms Pyruvatdecarboxylase aus *Acetobacter pasteurianus,* der *Ap*PDC-E469G, katalysiert, bei der in Position Nr. 469 Glutamat durch Glycin ausgetauscht ist. Der höchste Enantiomerenüberschuss an S-(PAC)-Derivaten, der dabei mit dem isolierten Enzym erreicht wurde, liegt bei 89 %, wie es von Rother Nee Gocke, Doerte; Kolter, Geraldine; Gerhards, Tina; Berthold, Catrine L.; Gauchenova, Ekaterina; Knoll, Michael; Pleiss, Juergen; Mueller, Michael; Schneider, Gunter; Pohl, Martina der Veröffentlichung in ChemCatChem, 2011, vol. 3, 10 p. 1587 - 1596 beschrieben wird. Allerdings ist die Ausbeute an (*S*)-PAC ausgehend von Benzaldehyd und Acetaldehyd mit nur 2%, bei einem enantiomeren Überschuss (ee) von 61%, sehr gering.

Ausgehend von dem Stand der Technik ist es die Aufgabe der Erfindung ein alternatives enzymatisches Verfahren zur asymmetrischen Synthese von (*S*)-Phenylacetylcarbinol zur Verfügung zu stellen, das einen hohen Enantiomerenüberschuss von (*S*)-Phenylacetylcarbinol ermöglicht und Ausbeuten in einem wirtschaftlich interessanten Maßstab erzielt. Bei einer Herstellung mit ganzen Zellen sollte der Enantiomerenüberschuss größer als 43 % sein.
Weiterhin sollen keine Nebenprodukte und keine Regioisomere gebildet werden. Dabei sollen preisgünstige Edukte eingesetzt werden können, die nicht chiral sind. Es soll eine asymmetrische Synthese von (*S*)-Phenylacetylcarbinol ermöglicht werden. Teure Trennung von chiralen Produktgemischen sollen verhindert werden.
Es soll ein Enzym bereitgestellt werden, mit dem (*S*)-Phenylacetylcarbinol aus Benzaldehyd und Pyruvat oder Acetaldehyd hergestellt werden kann sowie eine für das Enzym kodierende DNA.
Weiterhin soll ein Verfahren zur Herstellung des Enzyms zur Verfügung gestellt werden.
Die nach dem Stand der Technik vorliegenden Nachteile und Probleme sollen überwunden werden.
Es soll ein Verfahren zur Herstellung von (*S*)-Phenylacetylcarbinol zur Verfügung gestellt werden, das auch bei Einsatz von Rohzellextrakten oder ganzen Zellen hohe Enantiomerenüberschüsse ermöglicht.
Die Nachteile des Standes der Technik sollen kumulativ überwunden werden.

Ausgehend vom Oberbegriff des Anspruchs 1 und der nebengeordneten Ansprüche wird die Aufgabe erfindungsgemäß gelöst mit den im kennzeichnenden Teil der Ansprüche angegebenen Merkmalen. Die Aufgabe wird erfindungsgemäß gelöst durch eine Variante der Lyase *Ap*PDC-E469G bei der das Isoleucin in Position Nr. 468 durch eine Aminosäure ausgetauscht ist, die eine gegenüber Isoleucin verminderte Raumerfüllung hat, wobei sie eine Aminosäuresequenz nach einer der Sequenzen Nr. 1 oder 2 aufweist.

Diese Lyase hat einen positiven Einfluss auf die Erhöhung der Stereoselektivität bei der Herstellung von (*S*)-Phenylacetylcarbinol.

Weiterhin wird die Aufgabe gelöst durch erfindungsgemäße Varianten der Lyase *Ap*PDC-E469G bei der das Isoleucin in Position Nr. 468 durch eine Aminosäure ausgetauscht ist, die eine gegenüber Isoleucin verminderte Raumerfüllung hat und bei der die Aminosäure in Position Nr. 543 zusätzlich durch eine Aminosäure ausgetauscht ist, die eine kleinere Raumerfüllung hat als Tryptophan hat, wobei sie eine Aminosäuresequenz nach einer der Sequenzen Nr. 10 oder 23 hat.Weiterhin wird die Aufgabe dadurch gelöst, dass diese Lyasen für die Umsetzung von Benzaldehyd mit Pyruvat oder Acetaldehyd zu (*S*)-Phenylacetylcarbinol eingesetzt werden.

Mit den erfindungsgemäßen Lyasen sowie dem Herstellungsverfahren für (*S*)-Phenylacetylcarbinol ist es nunmehr möglich, (*S*)-Phenylacetylcarbinol bei Verwendung von ganzen Zellen von 93 % ee und bei Verwendung eines Rohzellextraks von 85 % ee herzustellen. Es werden keine Nebenprodukte, insbesondere keine Regioisomere gebildet. Dadurch, dass die Synthese mit unchiralen Edukten auskommt, ist sie preisgünstig. Auf eine Enantiomerentrennung kann verzichtet werden. Bei der Herstellung von (*S*)-Phenylacetylcarbinol können auch mit Rohzellextrakten oder ganzen Zellen hohe Enantiomerenüberschüsse erreicht werden. Alle nach dem Stand der Technik beschriebenen Nachteile werden kumulativ ausgeräumt.

Vorteilhafte Weiterbildungen der Erfindung sind in den Unteransprüchen angegeben.

Vorzugsweise können folgende Lyasen genannt werden, die diese Anforderung erfüllen:
- *Ap*PDC-E469G-I468G gemäß Sequenz Nr. 1 mit Glycin in Position Nr. 468
- *Ap*PDC-E469G-I468A gemäß Sequenz Nr. 2 mit Alanin in Position Nr. 468
- *Ap*PDC-E469G-I468V gemäß Sequenz Nr. 3 mit Valin in Position Nr. 468

Zur Verbesserung des Enantiomerenüberschusses können auch die Enzyme *Ap*PDC-E469G-I468L, *Ap*PDC-E469G-1468T, *Ap*PDC-E469G-I468C oder *Ap*PDC-E469G-I468S bei der Herstellung von (*S*)-Phenylacetylcarbinol eingesetzt werden.

Weiterhin werden erfindungsgemäß Desoxyribonukleinsäuren zur Verfügung gestellt, die für die angegebenen Enzyme kodieren.

Erfindungsgemäß handelt es sich um, Desoxyribonukleinsäuren, die für eine Variante des Enzyms *Ap*PDC-E469G kodieren und die in Position Nr. 1402 bis 1404 für eine Aminosäure kodieren, die gegenüber Isoleucin eine verminderte Raumerfüllung aufweist.

Für das Beispiel nach Sequenz Nr.1, bei dem in dieser Position für die Aminosäure Glycin kodiert wird, können sich in den Positionen Nr. 1402-1404 beispielsweise die Nukleinsäuren GGT befinden.

Beispielhaft ist eine für das Enzym *Ap*PDC-E469G-I468G mit Glycin in Position Nr. 468 nach Sequenz Nr.1 kodierende Desoxyribonukleinsäure nach Sequenz Nr. 4 beschrieben.

Für die erfindungsgemäßem Enzyme *Ap*PDC-E469G-I468A gemäß Sequenz Nr. 2 mit Alanin in Position Nr. 468 und *Ap*PDC-E469G-1468V gemäß Sequenz Nr. 3 mit Valin in Position Nr. 468 können durch Austausch der entsprechenden Nukleotide in Position Nr. 1402-1404 die dafür kodierenden Desoxyribonukleinsäuren zur Verfügung gestellt werden.

Bevorzugt handelt es sich um Desoxyribonukleinsäuren, die für eine der erfindungsgemäßen Varianten des Enzyms *Ap*PDC-E469G kodieren, bei der ausschließlich das Kodon in Position Nr. 1402-1404 durch ein Kodon ausgetauscht ist, welches für eine Aminosäure kodiert, die weniger raumerfüllend ist als Isoleucin, wobei sie eine Nukleotidsequenz nach einer der Sequenzen Nr. 4 oder 52 hat.

Eine für die erfindungsgemäßen Enzyme kodierende DNA kann nach dem Fachmann bekannten Verfahren durch gerichtete oder ungerichtete Mutagenese erzeugt werden. Bevorzugt ist dabei die gerichtete Mutagenese. Diese Verfahren sind dem Fachmann bekannt. Im speziellen Beschreibungsteil wird ein Beispiel für eine Herstellung für eine Ausführungsform der Erfindung konkret offenbart. Diese Verfahrensweise kann grundsätzlich auch für alle anderen offenbarten Desoxyribonukleinsäuren und Enzyme eingesetzt werden, so dass alle erfindungsgemäßen Enzyme und Desoxyribonukleinsäuren auf analogem Weg hergestellt werden können.

Die Desoxyribonukleinsäuren können in einen Vektor, vorzugsweise einem Plasmid ligiert sein.

Als Leervektoren können beispielsweise pET-20b(+), pET-21a-d(+), pET-22b(+), pET-23a-d(+), pET-24a-d(+), pET-25b(+), pET-26b(+), pET-27b(+), pET-28a(+), pET-29a-c(+), pET-30a-c(+), pET-31b(+), pET-34b(+), pET-35b(+), pET-36b(+), pET-37b(+), pET-38b(+) eingesetzt werden, in die die entsprechende erfindungsgemäße DNA ligiert werden.

Alternativ können die Desoxyribonukleinsäuren auch in das Genom ligiert sein.

Bei den ligierten Desoxyribonukleinsäuren handelt es sich um DNA-Sequenzen, die für eine Variante des Enzyms *Ap*PDC-E469G kodieren und die in Position Nr. 1402-1404 für eine Aminosäure kodieren, die gegenüber Isoleucin eine verminderte Raumerfüllung aufweist. Die Desoxyribonukleinsäuren können auch für die Emzyme *Ap*PDC-E469G-I468L, *Ap*PDC-E469G-I468T, *Ap*PDC-E469G-I468C oder *Ap*PDC-E469G-I468S kodieren.

Vorzugsweise kodiert die ligierte Desoxyribonukleinsäure für die Proteine nach den Sequenzen 1, 2, und 3. Besonders bevorzugt kodiert die ligierte Desoxyribonukleinsäure für die Proteine nach den Sequenzen 1 oder 2.

Erfindungsgemäß können Vektoren zur Verfügung gestellt werden, welche eine Desoxyribonukleinsäure enthält, die für eine Variante des Enzyms *Ap*PDC-E469G kodiert und die in Position Nr. 1402-1404 für eine Aminosäure kodiert, die gegenüber Isoleucin eine verminderte oder gleiche Raumerfüllung aufweist.

Vorzugsweise enthält der Vektor eine Desoxyribonukleinsäure nach Sequenz Nr.4 oder Sequenz Nr. 52.

Vorzugsweise ist der Vektor ein Plasmid.

Weiter bevorzugt ist ein erfindungsgemäße Vektor bei dem die erfindungsgemäßen Desoxyribonukleinsäuren in einem Leervektor aus der Gruppe pET-20b(+), pET-21a-d(+), pET-22b(+), pET-23a-d(+), pET-24a-d(+), pET-25b(+), pET-26b(+), pET-27b(+), pET-28a(+), pET-29a-c(+), pET-30a-c(+), pET-31b(+), pET-34b(+), pET-35b(+), pET-36b(+), pET-37b(+), pET-38b(+) ligiert sind.

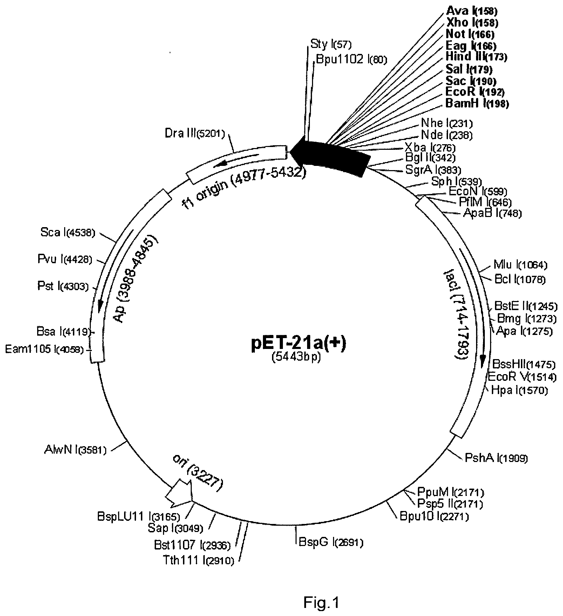

### Figur 1 zeigt ein erfindungsgemäßes Plasmid (pET-21 a(+) Vektorkarte).

In Sequenz Nr. 5 ist beispielhaft eine DNA-Sequenz für ein erfindungsgemäßes Plasmid dargestellt, die eine DNA nach Sequenz Nr. 4 enthält.

Erfindungsgemäß wird Benzaldehyd mit Pyruvat oder mit Acetaldehyd gemäß Formel (1) mittels einer Variante des Enzyms *Ap*PDC-E469G, die in Position Nr. 468 eine Aminosäure aufweist, die gegenüber Isoleucin eine verminderte Raumerfüllung aufweist, vorzugsweise ein Enzym aus der Gruppe nach Sequenz Nr. 1 oder 2, zu (*S*)-Phenylacetylcarbinol umgesetzt.

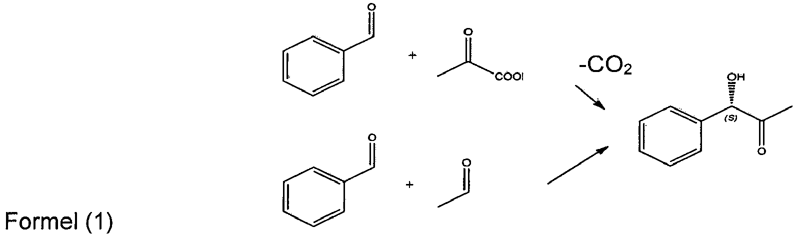

Die Umsetzung erfolgt vorzugsweise in wässriger Lösung.

Der pH-Wert liegt in einem Bereich von 5 - 9, vorzugsweise 6,5 - 8, besonders bevorzugt 6,5-7.

Dazu können als Puffer beispielsweise Kaliumphosphatpuffer, HEPES, MOPS, TEA oder TRIS-HCI eingesetzt werden.

Weiterhin können als Kofaktoren Thiamindiphosphat und Magnesiumsulfat eingesetzt werden.

Die Reaktion kann in *vivo* oder in *vitro* durchgeführt werden.

Für die in *vivo* Produktion von (*S*)-Phenylacetylcarbinol kann als Produktionsorganismus beispielsweise *E.coli,* ein *Corynebakterium,* beispielsweise *Corynebakterium glutamicum,* oder eine Hefe, wie *Saccheromycies cerevisiae* eingesetzt werden.

Dazu werden die Produktionsorganismen mit der erfindungsgemäßen DNA oder einem Vektor, der die DNA enthält transformiert.

Die DNA kann im Produktionsorganismus auch in das Genom eingeführt sein.

Die eingesetzten Gene werden dabei heterolog exprimiert.

Für die in *vitro* Produktion kann entweder das isolierte Enzym oder der Zellextrakt des Produktionsorganismus eingesetzt werden.

Typische Temperaturen liegen zwischen 20 °C und 40 °C, bevorzugt sind 20 °C bis 30 °C, besonders bevorzugt ist eine Temperatur von 20 °C bis 25 °C.

Die Reaktionszeiten können 2 h - 48 h, vorzugsweise 6 h - 24 h, besonders bevorzugt 12 h sein.

Im Folgenden werden einige Beispiele vorgestellt, die nicht beschränkend auszulegen sind.

Die Reaktionen können in einem klassischen Ansatz in einem Reaktionskolben unter Rühren durchgeführt werden.

Um (S)-PAC in hohen Enanantiomerenüberschüssen herstellen zu können, wurde eine Variante des Enzyms *Ap*PDC-E469G durch Mutagenese erzeugt. Die Variante, *Ap*PDC-E469G-I468G produziert (*S*)-PAC unter Verwendung des Rohzellextrakts Enzymen mit einem ee von 85 %. Unter Verwendung von *Ap*PDC-E469G-I468G, das heterolog in *Escherichia coli* exprimiert wurde und als kostengünstiger Ganzzellen-Katalysator eingesetzt wird, kann (*S*)-PAC mit einem ee von 93 % generiert werden.

### Ausführungsbeispiel 1:

20 mM Benzaldehyd, 400 mM Pyruvat, 2,5 mM Magnesiumsulfat, 100 µM Thiamindiphosphat, 20 mg/mL (Feuchtgewicht), *Ap*PDC-E469G-I468G (ganze Zellen von *E. coli* in denen *Ap*PDC-E469G-I468G exprimiert vorlag), 50 mM Kaliumphosphat-Puffer pH 6.5, 25 °C, Reaktionszeit: 48 h.

Enantiomerenreinheit von (*S*)-PAC: ee 93 %.
Ausbeute: 67 %.

### Ausführungsbeispiel 2:

20 mM Benzaldehyd, 400 mM Pyruvat, 2,5 mM Magnesiumsulfat, 100 µM Thiamindiphosphat, 1 mg/mL *Ap*PDC-E469G-I468G (Rohzellextrakt von *E*. *coli* Zellen in denen *Ap*PCD-E469G-I468G exprimiert vorlag), 50 mM Kaliumphosphat-Puffer pH 6.5, 25 °C, Reaktionszeit: 48 h.

Enantiomerenreinheit von (*S*)-PAC: ee 85 %.

In Folgenden wird die Herstellung der erfindungsgemäßen Enzyme und der dafür kodierenden Desoxyribonukleinsäuren beispielhaft erläutert. Das dargestellte Verfahren kann grundsätzlich auch für die Herstellung der anderen erfindungsgemäßen Lyasen und die dafür kodierenden Desoxyribonukleinsäuren analog eingesetzt werden.

### Herstellung der DNA der Enzymvariante ApPDC-E469G-I468G

### "Site saturated mutagenesis"

Die Methode des sogenannten "Site saturated mutagenesis" nach der Variante von Reets et. al (Reetz, Kahakeaw et al. 2008) wurde ausgehend von der Gensequenz *Ap*PDC-E469G (Template-DNA) durchgeführt, um Aminosäureaustausche an der Position Nr. I468 zu erhalten. Bei dieser Methode werden NDT-Kodons verwendet, die für 12 von 20 natürlichen Aminosäuren kodieren.

### Polymerasekettenreaktion (PCR)

In einem initialen Schritt wird die Template-DNA mittels Polymerasekettenreaktion (PCR) vervielfältigt und dabei gleichzeitig durch die Verwendung von sogenannten degenerierten Primer und den NDT-Kodons Mutationen eingefügt. Die verwendeten Primer wurden von der Firma "Eurofins MWG Operon" (http://www.eurofinsgenomics.eu) bestellt und hatten folgende Sequenz:

### Primer für die Site Saturated Mutagenesis zur Herstellung von ApPDC-E469G-I468NDT

| | |
|---|---|
| vorwärts: | 5' CCGTGGCTATGTCNDTGGCATCGCCATTC 3' (Seq. Nr. 6) |
| rückwärts: | 5' GAATGGCGATGCCAHNGACATAGCCACGG 3' (Seq. Nr. 7) |

Es wurde zunächst eine Master-Lösung hergestellt und anschließend in vier Ansätze zu je 50 µl aufgeteilt. Zur Start der Reaktion wurde 1 µl "KOD Hot Start Polymerase" hinzugegeben.

### PCR-Reaktionsansatz:

1-fach PCR-Puffer
5 % (v/v) DMSO
2 mM MgSO₄
0.2 mM Nukleotide
0.25 pmol vorwärts Primer
0.25 pmol rückwärts Primer
0.1 ng/µl DNA-Template

Die Reaktion wurde unter folgenden Bedingungen durchgeführt:

| | Dauer (min) | Temperatur (°C) | Wiederholungen |
|---|---|---|---|
| Initialisierung | 2:00 | 95 | 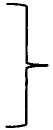 20x |
| Denaturierung | 0:20 | 95 | |
| Annealing | 1:00 | 75.5 | |
| Elongation | 6:00 | 70 | |
| Termination | 10:00 | 70 | |

Zum Verdau der Template-DNA wurde 1 µl des Enzyms Dpnl (Eppendorf) zur Lösung hinzugegeben und für 1 h bei 37 °C inkubiert. Der gesamte Ansatz wurde dann mit dem "DNA Purification Kit" (Chemikalienliste) vor der weiteren Transformation gereinigt.

### Transformation von E.coli BL21-DE3 und E.coli DH5a

Die Stämme *E.coli* BL21-DE3 und *E.coli* DH5a wurden mit der durch **"Site** Saturation Mutagenesis" hergestellte DNA transformiert. Dazu wurde zu 50 µl kompetenten Zellen 100 ng der DNA hinzugegeben und auf Eis für 30 min inkubiert. Anschließend erfolgte ein Hitzeschock für 90 sek. bei 42 °C. Nach 3 min. auf Eis wurden 500 µl SOC-medium hinzugegeben und die Lösung am Anschluss für 45 min bei 350 UPM und 37 °C im Eppendorf Thermomixer inkubiert. Nach erfolgter Inkubation wurde die Zellsuspension bei 13.000 UPM in einer Eppendorf Tischzentrifuge für 30 sek zentrifugiert und das Pellet daraufhin in 100 µl Überstand resuspendiert. Die auf 100 µl eingeengte Zellsuspension wurde auf LB-Aggarplatten (mit 100 µg/ml Ampicillin) ausplattiert und für 16 h bei 37 °C über Kopf inkubiert.

### Expression der Enzymvarianten

46 Einzelkolonien der Transformation wurden von der Platte mit einem Zahnstocher gepickt und für 24 h bei 20 °C und 850 UPM in jeweils einem *well* einer 48er-Nerbe-Platte (Nerbe Plus GmbH) mit je 1 ml LB-Medium inkubiert ("Masterplate"). Ein weiteres *well* wurde mit *E.coli* BL 21-DE3 Zellen inokuliert, die zuvor analog mit der *ApPDC*-E469G-Template-DNA transformiert wurden. Nach erfolgter Inkubation wurden zu je 1.5 ml Autoinduktionsmedium 10 µl der Zellsuspensionen in 48-well-FlowerPlates® (m2p-labs, Germany) gegeben. Die FlowerPlate wurde für 48 h bei 20 °C und 850 UPM inkubiert. Das restliche Volumen (990 µl) der "Masterplate" wurde mit 300 µl Glycerin versetzt und bei -80 °C gelagert.

### Zellaufschluss und Carboligation

Die in den FlowerPlates® exprimierten Varianten wurden durch Einfrieren (48 h, 4°C) aufgeschlossen. Nach dem Wiederauftauen wurden je 500 µl der Zellsuspensionen in zwei *wells* einer 96-we*ll*-Platte überführt (Doppelbestimmung). Die Platte wurde für 3 min bei 4.000 UPM zentrifugiert und das Pellet in 420 µl KPi-Puffer mit 1 mg/ml Lysozym resuspendiert. Die Platte wurde für 1 h bei 20 °C und 400 UPM inkubiert und anschließend erneut für 10 min bei 4.000 UPM zentrifugiert. Jeweils 250 µl des Überstands wurde in jeweils ein *well* einer 2 ml-Nerbe-Platte pipettiert und 250 µl einer Reaktionslösung aus 40 mM Benzaldehyd, 400 mM Pyruvat, 4 mM Magnesiumsulfat und 400 µM Thiamindiphosphat hinzugegeben. Die Platte wurde erneut für 24 h inkubiert und die Reaktionslösungen anschließend analysiert (siehe HPLC-Analyse).

### HPLC-Analyse

200 µl der Carboligation-Reaktionslösungen wurden mit jeweils 200 µl Heptan versetzt, gevortext und anschließend jeweils 150 µl der oberen Phase in HPLC-Vials überführt. Die Analyse der Proben erfolgte mit einer Chiralpak IC-3 Säule (Chiral Technologies Inc.) unter Verwendung der folgenden Methode.

### HPLC Programm

| | |
|---|---|
| Länge | 24 min |
| Fluss | 0.5 ml/min |
| mobile Phase | 25 % Isopropanol |
| | 75 % Heptan |

### Typische Retentionszeiten und verwendete Wellenlänge zur Quantifizierung

| | Retentionszeit (min) | Wellenlänge (nm) |
|---|---|---|
| (R)-PAC | 12.3 | 210 |
| (S)-PAC | 12.9 | 210 |
| Benzaldehyd | 11.4 | 254 |

### DNA-Isolierung und Identifizierung der besten Enzymvarianten durch DNA-Sequenzierung

Die DNA des Enzyms, das in den Carboligationsreaktionen den höchsten ee-Werten für (S)-PAC lieferte, wurde ausgehend von der Masterplatte zur Identifizierung der Mutation sequenziert. Dazu wurde zunächst Zellen mit einer Impföse aus der mit Glycerin versetzten Masterplatte 50 mL LB-Medium (+50 µg/ml Ampicillin) überführt und in einem 250 mL Erlenmeyerkolben bei 37 °C inkubiert. Nach 12 h Inkubation wurden 20 mL der Zellsuspension zentrifugiert (4.000 UPM, 5 min, 4 °C). Die DNA der Zellen im Pellet wurde analog zu den Herstellerangaben (Qiagen N.V.) nach der Methode des "QIAprep® Spin Miniprep Kit" isoliert. Die Konzentration der DNA wurde dazu auf 100 ng/µl eingestellt und durch die Firma LGC Genomics GmbH sequenziert.

### LB (Lysogeny Broth)-Medium

| | | |
|---|---|---|
| 10 g/l | NaCl | |
| 10 g/l | Peptone | |
| 5 g/l | Yeast extract | 20 |

### Alternative, gerichtete Methode zur Herstellung der Variante ApPDC-E469G-I468G mittels QuikChange®

Eine andere Methode zur Herstellung der Enzymvariante *Ap*PDC-E469G/I468G wäre beispielsweise die sogenannte QuikChange®-PCR-Methode (U.S. Patent Nr. 5.789.166, 5.932.419, 6.391.548). Bei dieser Variante der PCR wird ein Primerpaar verwendet, das an der Stelle des auszutauschenden DNA-Triplettcodes die entsprechende Sequenzveränderung trägt. Zur Herstellung der Enzymvariante *Ap*PDC-E469G/I468G kann das Gen verwendet werden, das für die Variante *Ap*PDC-E469G kodiert. Dieses so genannte DNA-Template sollte in einem Vektor (beispielsweise pET22a) kloniert vorliegen. Anstelle des Triplett-Codes, der in Position I468 für die Aminosäure Tryptophan kodiert, muss ein Primer verwendet werden, der die für Glycin kodierende Mutation an dieser Position trägt (also: GGA, GGT, GGC oder GGG). Alle weiteren Parameter dieser QuikChange®-PCR-Methode sowie die Auswahl der benötigten Primer können analog zu den Angaben des Herstellers (Agilent Technologies, Inc.) mittels der Anleitung des "QuikChange® Site-Directed Mutagenesis Kit" durchgeführt werden.

### DNA-Template (ApPDC-E469G) der QuikChange®-PCR-Methode zur Herstellung der Variante ApPDC-E469G-I468G

Im Sequenzprotokoll ist die Sequenz mit der Seq. Nr. 8 dargestellt. Sequenz Nr. 8 ist hier beispielhaft für eine DNA offenbart, die für das zu verändernde Protein *Ap*PDC-E469G nach Sequenz Nr. 53 kodiert. Erfindungsgemäß können jedoch alle anderen, für das zu verändernde Ausgangsprotein kodierenden Desoxyribonukleinsäuren, für die Herstellung des zu verändernden Enzyms eingesetzt werden. Die dafür kodierenden Nukleotide sind dem Fachmann bekannt.

### Herstellung der Varianten in Form von "ganzen Zellen"

Zur Expression der Enzyme in ganzen Zellen im 1L-Maßstab wurden zunächst Zellen von der mit Glycerin versetzten Masterplatte mit einer Impföse in 50 mL LB-Medium (+100 µg/ml Ampicillin) überführt und in einem 250 mL Erlenmeyerkolben bei 120 UPM und 37 °C inkubiert. Nach 16 h Inkubation wurden die zu 1 L Autoinduktionsmedium 10 mL der Kultur gegeben und in einem 5 L Erlenmeyerkolben für 72 h bei 90 UPM und 20 °C inkubiert. Die Zellen wurden anschließend durch Zentrifugation (4 °C, 6.000 UPM, 30 min) geerntet und bis zur weiteren Verwendung bei -20 °C gelagert.

### Autoinduktionsmedium

| | |
|---|---|
| 12 g/l | Pepton |
| 24 g/l | Hefeextrakt |
| 90 mM | Kaliumphosphat-Puffer (pH 7,5) |
| 0,5 g/l | Glucose |
| 2 g/l | Lactose |
| 0,01 g/l | Ampicillin |
| 6.3 g/l | Glycerin |

### Herstellung der Varianten in Form von "Zellextrakten"

10 g der im 1L-Maßstab kultivierten Zellen (siehe "Herstellung der Varianten in Form von **"ganzen** Zellen"") wurden mit 25 mL Aufschlusspuffer (50 mM Kaliumphosphat pH 6.5, 100 µM Thiamindiphosphat, 2 mM Magnesiumsulfat), der auf 4 °C gekühlt war, auf Eis resuspendiert. Anschließend wurden die resuspendierten Zellen mittels Ultraschall (SD14-Sonotrode (Hielscher Ultrasonics GmbH), 4x2 min Beschall mit je 1 min Abkühlung aus Eis) aufgeschlossen. Zur Abtrennung von Zelltrümmern wurde die Lösung zentrifugiert (45 min, 18.000 UPM, 4 °C) und der Überstand ("Zellextrakt") in ein neues Gefäß überführt.

### Herstellung der Varianten in Form von "isolierten Enzymen"

Zur Reinigung der *Ap*PDC-Variante mittels immobilisierte Metallionen-Affinitätschromatographie und Größenausschlusschromatographie wurde ein "ÄKTA™purifier" der Firma "Amersham Bioscience" verwendet, um u.a. die Protein-UV-Absorption (280 nm) sowie die elektrische Leitfähigkeit zu detektieren und die Flussrate einzustellen. Zur Reinigung wurden 25 mL der hergestellte Zellextrakt (siehe "Herstellung der Varianten in Form von "Zellextrakten"") mit einem Flussrate von 3 mL/min auf eine Säule mit einem Volumen von 60 mL Ni-NTA-Superflow (Qiagen N.V.) aufgetragen, die zuvor mit 180 mL des Auftragspuffer equilibriert wurde. Im Anschluss wurde weiter mit Auftragspuffer in einer Flussrate von 5 ml/min gespült, um nicht-, bzw. sehr schwach an das Säulenmaterial bindende Proteine zu entfernen. Nachdem die UV-Absorption (280 nm) wieder eine stabile Basisline erreicht hatte, wurde ein Waschpuffer (50 mM Kaliumphosphat pH 6.5, 100 µM Thiamindiphosphat, 2 mM Magnesiumsulfat, 50 mM Imidazol) mit einer Flussrate von 5 mL/min zur Elution von schwach an das Säulenmaterial bindenden Proteinen verwendet. Nach erneut stabiler UV-Absorption (280 nm) wurde zur Elution des Zielproteins ein Elutionspuffer (50 mM Kaliumphosphat pH 6.5, 100 µM Thiamindiphosphat, 2 mM Magnesiumsulfat, 250 mM Imidazol) mit einer Flussrate von 5 mL/min verwendet.

Das Eluat der IMAC wurde zur Umpufferung mit einer Flussrate von 10 mL/min auf eine Größenausschlusschromatographie-Säule (1 L Säulenvolumen, Sephadex-G25, GE-Healthcare) aufgetragen, die zuvor mit 2 L Umpufferungspuffer (10 mM Kaliumphosphat pH 6.5, 100 µM Thiamindiphosphat, 2 mM Magnesiumsulfat) gespült wurde. Die Fraktionen mit erhöhter UV-Absorption (280 nm) wurden vereinigt und in einer Kristallisationsschale eingefroren (-20 °C). Zur Gefriertrocknung wurde an die eingefrorene Proteinlösung für 3 Tage ein Unterdruck von 0.22 mBar angelegt.

In einer weiteren vorteilhaften Ausgestaltung der Erfindung ist bei dem erfindungsgemäßen Enzym zusätzlich zu dem erfindungsgemäßen Austausch in Position Nr. 468 die Aminosäure Tryptophan in Position Nr. 543 durch eine Aminosäure ausgetauscht, die eine kleinere Raumerfüllung als Tryptophan besitzt.

Diese Lyasen haben zusätzlich noch einen positiven Einfluss auf die Erhöhung der Stereoselektivität bei der Herstellung von (S)-Phenylacetylcarbinol.

Mit dieser bevorzugten Ausführungsform der Erfindung kann ein Enantiomerenüberschuss von 98 % ee erzielt werden.

Vorzugsweise können folgende Lyasen genannt werden, die diese Anforderung erfüllen:
- *Ap*PCD-E469G-I468G-W543H gemäß Sequenz Nr. 9 mit Histidin in Position Nr. 543.
- *Ap*PCD-E469G-I468G-W543F gemäß Sequenz Nr. 10 mit Phenylalanin in Position Nr. 543.
- *Ap*PCD-E469G-I468G-W543P gemäß Sequenz Nr. 11 mit Prolin in Position Nr. 543.
- *Ap*PCD-E469G-I468G-W543I gemäß Sequenz Nr. 12 mit Isoleucin in Position Nr. 543.
- *Ap*PCD-E469G-I468G-W543L gemäß Sequenz Nr.13 mit Leucin in Position Nr. 543.
- *Ap*PCD-E469G-I468G-W543M gemäß Sequenz Nr.14 mit Methionin in Position Nr. 543.
- *Ap*PCD-E469G-I468G-W543V gemäß Sequenz Nr. 15 mit Valin in Position Nr. 543.
- *Ap*PCD-E469G-I468G-W543A gemäß Sequenz Nr. 16 mit Alanin in Position Nr. 543.
- *Ap*PCD-E469G-I468G-W543Y gemäß Sequenz Nr. 17 mit Tyrosin in Position Nr. 543.
- *Ap*PCD-E469G-I468G-W543T gemäß Sequenz Nr.18 mit Threonin in Position Nr. 543.
- *Ap*PCD-E469G-I468G-W543G gemäß Sequenz Nr. 19 mit Glycin in Position Nr. 543.
- *Ap*PCD-E469G-I468G-W543S gemäß Sequenz Nr. 20 mit Serin in Position Nr. 543.
- *Ap*PCD-E469G-I468G-W543C gemäß Sequenz Nr. 21 mit Cystein in Position Nr. 543.
   sowie
- *Ap*PCD-E469G-I468A-W543H gemäß Sequenz Nr. 22 mit Histidin in Position Nr. 543.
- *Ap*PCD-E469G-I468A-W543F gemäß Sequenz Nr. 23 mit Phenylalanin in Position Nr. 543.
- *Ap*PCD-E469G-I468A-W543P gemäß Sequenz Nr. 24 mit Prolin in Position Nr. 543.
- *Ap*PCD-E469G-I468A-W543I gemäß Sequenz Nr. 25 mit Isoleucin in Position Nr. 543.
- *Ap*PCD-E469G-I468A-W543L gemäß Sequenz Nr. 26 mit Leucin in Position Nr. 543.
- *Ap*PCD-E469G-I468A-W543M gemäß Sequenz Nr. 27 mit Methionin in Position Nr. 543.
- *Ap*PCD-E469G-I468A-W543V gemäß Sequenz Nr. 28 mit Valin in Position 543.
- *Ap*PCD-E469G-I468A-W543A gemäß Sequenz Nr. 29 mit Alanin in Position Nr. 543.
- *Ap*PCD-E469G-I468A-W543Y gemäß Sequenz Nr. 30 mit Tyrosin in Position Nr. 543.
- *Ap*PCD-E469G-I468A-W543T gemäß Sequenz Nr. 31 mit Threonin in Position Nr. 543.
- *Ap*PCD-E469G-I468A-W543G gemäß Sequenz Nr. 32 mit Glycin in Position Nr. 543.
- *Ap*PCD-E469G-I468A-W543S gemäß Sequenz Nr. 33 mit Serin in Position Nr. 543.
- *Ap*PCD-E469G-I468A-W543C gemäß Sequenz Nr. 34 mit Cystein in Position Nr. 543. und
- *Ap*PCD-E469G-I468V-W543H gemäß Sequenz Nr. 35 mit Histidin in Position Nr. 543.
- *Ap*PCD-E469G-I468V-W543F gemäß Sequenz Nr. 36 mit Phenylalanin in Position Nr. 543.
- *Ap*PCD-E469G-I468V-W543P gemäß Sequenz Nr. 37 mit Prolin in Position Nr. 543.
- *Ap*PCD-E469G-I468V-W543I gemäß Sequenz Nr. 38 mit Isoleucin in Position Nr. 543.
- *Ap*PCD-E469G-I468V-W543L gemäß Sequenz Nr. 39 mit Leucin in Position Nr. 543.
- *Ap*PCD-E469G-I468V-W543M gemäß Sequenz Nr. 40 mit Methionin in Position Nr. 543.
- *Ap*PCD-E469G-I468V-W543V gemäß Sequenz Nr. 41 mit Valin in Position Nr. 543
- *Ap*PCD-E469G-I468V-W543A gemäß Sequenz Nr. 42 mit Alanin in Position Nr. 543.
- *Ap*PCD-E469G-I468V-W543Y gemäß Sequenz Nr. 43 mit Tyrosin in Position Nr. 543.
- *Ap*PCD-E469G-I468V-W543T gemäß Sequenz Nr. 44 mit Threonin in Position Nr. 543.
- ApPCD-E469G-I468V-W543G gemäß Sequenz Nr. 45 mit Glycin in Position Nr. 543.
- *Ap*PCD-E469G-I468V-W543S gemäß Sequenz Nr. 46 mit Serin in Position Nr. 543.
- *Ap*PCD-E469G-I468V-W543C gemäß Sequenz Nr. 47 mit Cystein in Position Nr. 543.

Erfindungsgemäß wird eine Lyase zur Verfügung gestellt, bei der die Aminosäure in Position Nr. 543 zusätzlich durch eine Aminosäure ausgetauscht ist, die eine kleinere Raumerfüllung hat als Tryptophan, wobei sie eine Aminosäuresequenz nach einer der Sequenzen Nr. 10 oder 23 hat.

In einer besonders bevorzugten Variante wird erfindungsgemäß eine Lyase zur Verfügung gestellt, bei der die Aminosäure in Position Nr. 543 zusätzlich durch eine Aminosäure ausgetauscht ist, die eine kleinere Raumerfüllung hat als Tryptophan, wobei sie eine Aminosäuresequenz nach Sequenz Nr. 23 hat.

Weiterhin werden erfindungsgemäß Desoxyribonukleinsäuren zur Verfügung gestellt, die für die angegebenen Enzyme kodieren.

Erfindungsgemäß handelt es sich um Desoxyribonukleinsäuren, die für eine Variante des Enzyms *Ap*PDC-E469G kodieren und die in Position Nr. 1402-1404 für eine Aminosäure kodieren, die gegenüber Isoleucin eine verminderte Raumerfüllung aufweist und die zusätzlich in Position Nr. 1627-1629 für eine Aminosäure kodieren, die eine kleinere Raumerfüllung ausweist als Tryptophan.

Für das Beispiel des Proteins nach Sequenz Nr. 23 können sich in den Positionen Nr. 1402-1404 der entsprechenden Desoxyribonukleinsäure beispielsweise die Nukleinsäuren GCC befinden und in den Positionen Nr. 1627-1629 die Nukleinsäuren TTT befinden. Die entsprechende Nukleotidsequenz ist im Sequenzprotokoll unter der Nummer 48 gelistet.

Erfindungsgemäß wird eine Desoxyribonukleinsäure zur Verfügung gestellt, kodierend für eine Variante des Enzyms *Ap*PDC-E469G, bei der ausschließlich das Kodon in Position Nr. 1402-1404 durch ein Kodon ausgetauscht ist, welches für eine Aminosäure kodiert, die weniger raumerfüllend ist als Isoleucin und bei der zusätzlich das Kodon in Position Nr. 1627 bis 1629 durch ein Kodon ausgetauscht ist, welches für eine Aminosäure kodiert, die weniger raumerfüllend ist als Tryptophan, wobei sie eine Nukleotidsequenz nach Sequenz Nr. 48 hat.

Eine für die erfindungsgemäßen Enzyme nach den Sequenzen 9 bis 47 kodierende DNA kann nach dem Fachmann bekannten Verfahren durch gerichtete oder ungerichtete Mutagenese erzeugt werden. Bevorzugt ist dabei die gerichtete Mutagenese. Diese Verfahren sind dem Fachmann bekannt. Im speziellen Beschreibungsteil wird ein Beispiel für eine Herstellung für eine Ausführungsform der Erfindung konkret offenbart. Diese Verfahrensweise kann grundsätzlich auch für alle anderen offenbarten Desoxyribonukleinsäuren und Enzyme eingesetzt werden, so dass alle erfindungsgemäßen Enzyme und Desoxyribonukleinsäuren auf analogem Weg hergestellt werden können.

Die Desoxyribonukleinsäuren können in einen Vektor, vorzugsweise einem Plasmid ligiert sein.

Als Leervektoren können beispielsweise pET-20b(+), pET-21a-d(+), pET-22b(+), pET-23a-d(+), pET-24a-d(+), pET-25b(+), pET-26b(+), pET-27b(+), pET-28a(+), pET-29a-c(+), pET-30a-c(+), pET-31b(+), pET-34b(+), pET-35b(+), pET-36b(+), pET-37b(+), pET-38b(+) eingesetzt werden, in die die entsprechende erfindungsgemäße DNA ligiert wird.

Alternativ können die Desoxyribonukleinsäuren auch in das Genom ligiert sein.

Bei den ligierten Desoxyribonukleinsäuren handelt es sich um DNA, die für eine Variante des Enzyms *Ap*PDC-E469G kodieren und die in Position Nr. 1402-1404 für eine Aminosäure kodiert, die gegenüber Isoleucin eine verminderte Raumerfüllung aufweist und die in Position Nr. 1627-1629 für eine Aminosäure kodiert, die gegenüber Tryptophan eine verminderte Raumerfüllung aufweist.

Vorzugsweise kodiert die ligierte Desoxyribonukleinsäure für die Proteine nach den Sequenzen 9 bis 47.

Erfindungsgemäß können Vektoren zur Verfügung gestellt werden, welche eine Desoxyribonukleinsäure enthält, die für eine Variante des Enzyms *Ap*PDC-E469G kodiert und die in Position Nr. 1402-1404 für eine Aminosäure kodiert, die gegenüber Isoleucin eine verminderte Raumerfüllung aufweist und die in Position Nr. 1627-1629 für eine Aminosäure kodiert, die gegenüber Tryptophan eine verringerte Raumerfüllung aufweist.

Vorzugsweise enthält der Vektor eine Desoxyribonukleinsäure nach Sequenz Nr. 48.

Vorzugsweise ist der Vektor ein Plasmid.

Weiter bevorzugt ist ein erfindungsgemäße Vektor bei dem die erfindungsgemäßen Desoxyribonukleinsäuren in einem Leervektor aus der Gruppe pET-20b(+), pET-21a-d(+), pET-22b(+), pET-23a-d(+), pET-24a-d(+), pET-25b(+), pET-26b(+), pET-27b(+), pET-28a(+), pET-29a-c(+), pET-30a-c(+), pET-31b(+), pET-34b(+), pET-35b(+), pET-36b(+), pET-37b(+), pET-38b(+) ligiert sind.

Eine bevorzugte Variante eines erfindungsgemäßen Vektors beinhaltet eine Desoxyribonukleinsäure ausgewählt aus der Gruppe enthaltend die Sequenzen Nr. 4, 48 und 52.

In Sequenz Nr. 49 ist beispielhaft eine Sequenz für ein erfindungsgemäßes Plasmid dargestellt, die eine DNA nach Sequenz Nr. 48 enthält.

Erfindungsgemäß wird auch in der vorteilhaften Ausgestaltung der Erfindung Benzaldehyd mit Pyruvat oder mit Acetaldehyd gemäß Formel (1) mittels einer Variante des Enzyms *Ap*PDC-E469G, das in Position Nr. 468 eine Aminosäure aufweist, die gegenüber Isoleucin eine verminderte Raumerfüllung aufweist und das in Position Nr. 543 eine Aminosäure aufweist, die gegenüber Tryptophan eine verringerte Raumerfüllung besitzt, vorzugsweise einem Enzym aus der Gruppe nach Sequenz Nr. 9 bis 47 zu (*S*)-Phenylacetylcarbinol umgesetzt.

Erfindungsgemäß bevorzugt wird ein Verfahren zur Herstellung von (*S*)- Phenylacetylcarbinol, bei dem in der vorteilhaften Ausgestaltung der Erfindung Benzaldehyd mit Pyruvat oder mit Acetaldehyd gemäß Formel (1) zu (*S*)- Phenylacetylcarbinol umgesetzt wird und zwar mittels einer Variante des Enzyms *Ap*PDC-E469G, das in Position Nr. 468 eine Aminosäure aufweist, die gegenüber Isoleucin eine verminderte Raumerfüllung aufweist und das in Position Nr. 543 eine Aminosäure aufweist, die gegenüber Tryptophan eine verringerte Raumerfüllung besitzt, wobei die Umsetzung mit einer Lyase gemäß einer Aminosäuresequenz ausgewählt aus der Gruppe enthaltend die Sequenzen Nr. 10 und 23 durchgeführt wird. Erfindungsgemäß umfasst ist somit auch ein Verfahren zur Herstellung von (*S*)- Phenylacetylcarbinol, bei dem in der vorteilhaften Ausgestaltung der Erfindung Benzaldehyd mit Pyruvat oder mit Acetaldehyd gemäß Formel (1) zu (*S*)- Phenylacetylcarbinol umgesetzt wird und zwar mittels einer Variante des Enzyms *Ap*PDC-E469G, das in Position Nr. 468 eine Aminosäure aufweist, die gegenüber Isoleucin eine verminderte Raumerfüllung aufweist und das in Position Nr. 543 eine Aminosäure aufweist, die gegenüber Tryptophan eine verringerte Raumerfüllung besitzt, wobei die Umsetzung mit einer Lyase gemäß einer Aminosäuresequenz ausgewählt aus der Gruppe enthaltend die Sequenzen Nr. 1, 2, 10 und 23 durchgeführt wird.

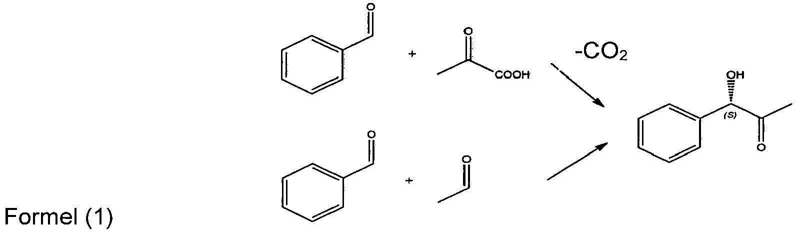

Die Umsetzung erfolgt vorzugsweise in wässriger Lösung.

Der pH-Wert liegt in einem Bereich von 5 - 9, vorzugsweise 6,5 - 8, besonders bevorzugt 6,5-7.

Dazu können als Puffer beispielsweise Kaliumphosphatpuffer, HEPES, MOPS, TEA oder TRIS-HCI eingesetzt werden.

Weiterhin können als Kofaktoren Thiamindiphosphat und Magnesiumsulfat eingesetzt werden.

Die Reaktion kann in *vivo* oder in *vitro* durchgeführt werden. Bevorzugt wird das erfindungsgemäße Verfahren *in vivo* durchgeführt.

Für die in *vivo* Produktion von (S)-Phenylacetylcarbinol kann als Produktionsorganismus beispielsweise *E.coli,* ein *Corynebakterium,* beispielsweise *Corynebakterium glutamicum,* oder eine Hefe, wie *Saccheromycies cerevisiae* eingesetzt werden.

Dazu werden die Produktionsorganismen mit der erfindungsgemäßen DNA oder einem Vektor, der die DNA enthält transformiert.

Die DNA kann im Produktionsorganismus auch in das Genom eingeführt sein.

Die eingesetzten Gene werden dabei heterolog exprimiert.

Für die in *vitro* Produktion kann entweder das isolierte Enzym oder der Zellextrakt des Produktionsorganismus eingesetzt werden.

Eine vorteilhafte Variante der vorliegenden Erfindung ist ein Verfahren, bei dem die Organismen mit mindestens einer DNA ausgewählt aus der Gruppe enthaltend die Sequenzen Nr. 4, Nr. 48 und Nr. 52 transformiert werden und/oder die DNA ausgewählt aus der Gruppe enthaltend die Sequenzen Nr. 4, Nr. 48 und Nr. 52 in das Genom integriert wird.

Eine weitere vorteilhafte Variante der vorliegenden Erfindung ist ein Verfahren bei dem für die Transformation ein erfindungsgemäßer Vektor eingesetzt wird.

Eine weitere vorteilhafte Variante der vorliegenden Erfindung ist ein Verfahren bei dem die Umsetzung in *vitro* mit Rohextrakt aus einem Produktionsorganismus oder isoliertem Enzym mit einer Aminosäuresequenz ausgewählt aus der Gruppe enthaltend die Sequenzen Nr. 1, 2, 10 und 23 durchgeführt wird.

Typische Temperaturen liegen zwischen 20 °C und 40 °C, bevorzugt sind 20 °C bis 30 °C, besonders bevorzugt ist eine Temperatur von 20 °C bis 25 °C.

Die Reaktionszeiten können 2 h - 48 h, vorzugsweise 6 h - 24 h, besonders bevorzugt 12 h sein.

Im Folgenden werden einige Beispiele vorgestellt, die nicht beschränkend auszulegen sind.

Die Reaktionen können in einem klassischen Ansatz in einem Reaktionskolben unter Rühren durchgeführt werden.

Um (*S*)-PAC in hohen Enanantiomerenüberschüssen herstellen zu können, wurde eine Variante des Enzyms *Ap*PDC-E469G durch Mutagenese erzeugt. Die Variante, *Ap*PDC-E469G-I468A-W543F produziert (*S*)-PAC unter Verwendung von isolierten Enzymen mit einem ee von 98 %. Unter Verwendung von *Ap*PDC-E469G-I468A-W543F, das heterolog in Escherichia coli exprimiert wurde und als kostengünstiger Ganzzellen-Katalysator eingesetzt wird, kann (*S*)-PAC mit einem ee von 96 % generiert werden.

### Ausführungsbeispiel 3:

20 mM Benzaldehyd, 400 mM Pyruvat, 2,5 mM Magnesiumsulfat, 100 µM Thiamindiphosphat, 20 mg/mL (Feuchtgewicht) *Ap*PDC-E469G-I468A-W543F (ganze Zellen von *E. coli* in denen *Ap*PDC-E469G-I468A-W543F exprimiert vorlag), 50 mM Kaliumphosphat-Puffer pH 6.5, 25 °C, Reaktionszeit: 48 h.

Enantiomerenreinheit von (S)-PAC: ee 96 %.
Ausbeute: 73 %.

### Ausführungsbeispiel 4:

20 mM Benzaldehyd, 400 mM Pyruvat, 2,5 mM Magnesiumsulfat, 100 µM Thiamindiphosphat, 1 mg/mL *Ap*PDC-E469G-I468A-W543F 20 mM Benzaldehyd, 400 mM Pyruvat, 2,5 mM Magnesiumsulfat, 100 µM Thiamindiphosphat, 1 mg/mL *Ap*PDC-E469G-I468G (isoliertes Enzym), 50 mM Kaliumphosphat-Puffer pH 6.5, 25 °C, Reaktionszeit: 48 h., 50 mM Kaliumphosphat-Puffer pH 6.5, 25 °C, Reaktionszeit: 48 h.

Enantiomerenreinheit von (S)-PAC: ee 98 %.
Ausbeute: 45 %.

### Gerichtete Methode zur Herstellung der Variante ApPDC-E469G-I468A-W543F mittels QuikChange® ausgehend von ApPDC-E469G-I468A-W543

Zur Herstellung der Enzymvariante *Ap*PDC-E469G-I468A-W543F wurde die sogenannte QuikChange®-PCR-Methode (U.S. Patent Nr. 5.789.166, 5.932.419, 6.391.548) verwendet. Bei dieser Variante der PCR wird ein Primerpaar genutzt, das an der Stelle des auszutauschenden DNA-Triplettcodes die entsprechende Sequenzveränderung trägt. Zur Herstellung der Enzymvariante *Ap*PDC-E469G-I468A-W543F wurde das Gen verwendet werden, das für die Variante *Ap*PDC-E469G-I468A-W543 kodiert. Dieses so genannte DNA-Template lag in einem Vektor (beispielsweise pET22a) kloniert vor. Anstelle des Triplett-Codes, der in Position W543 für die Aminosäure Tryptophan kodiert, muss ein Primer verwendet werden, der die für Phenylalanin kodierende Mutation an dieser Position trägt (also beispielsweise TTC oder TTT).

### Primer der QuikChange®-PCR-Methode zur Herstellung der Enzymvariante ApPDC-E469G-I468A-W543F ausgehend von ApPDC-E469G-I468A-W543

| | |
|---|---|
| vorwärts: | 5' ACCTTGCGGCCGAATTGAACCAGCATATCCGTGC 3' (Seq. Nr.50) |
| rückwärts: | 5' GCACGGATATGCTGGTTCAATTCGGCCGCAAGGT 3' (Seq. Nr. 51) |

Es wurde zunächst eine Master-Lösung hergestellt und anschließend in vier Ansätze zu je 50 µl aufgeteilt. Zur Start der Reaktion wurde 1 µl "*PfuTurbo*® DNA polymerase" hinzugegeben.

### PCR-Reaktionsansatz:

| | |
|---|---|
| 1 | -fachPCR-Puffer |
| 0.2 | mM Nukleotide |
| 0.25 | pmol vorwärts Primer |
| 0.25 | pmol rückwärts Primer |
| 0.1 | ng/µl DNA-Template |

Die Reaktion wurde unter folgenden Bedingungen durchgeführt:

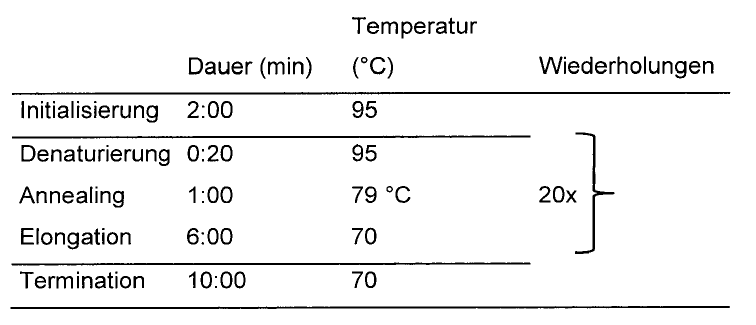

Zum Verdau der Template-DNA wurde 1 µl des Enzyms Dpnl (Eppendorf) zur Lösung hinzugegeben und für 1 h bei 37°C inkubiert. Der gesamte Ansatz wurde dann mit dem "DNA Purification Kit" (Chemikalienliste) vor der weiteren Transformation gereinigt.
Alle weiteren Parameter dieser QuikChange®-PCR-Methode sowie die Auswahl der benötigten Primer für weitere Enzymvarianten können analog zu den Angaben des Herstellers (Agilent Technologies, Inc.) mittels der Anleitung des "QuikChange® Site-Directed Mutagenesis Kit" verwendet werden.

### DNA-Template (ApPDC-E469G-I468A) der QuikChange®-PCR-Methode zur Herstellung der Variante ApPDC-E469G-I468A-W543F

Sequenz Nr. 52 ist hier beispielhaft für eine DNA offenbart, die für das zu verändernde Protein *Ap*PDC-E469G-I468A nach Sequenz Nr. 2 kodiert. Erfindungsgemäß können jedoch alle anderen, für das zu verändernde Ausgangsprotein kodierenden Desoxyribonukleinsäuren, für die Herstellung des zu verändernden Enzyms eingesetzt werden. Die dafür kodierenden Nukleotide sind dem Fachmann bekannt.

### Transformation von E.coli BL21-DE3 und E.coli DH5a

Die Stämme *E.coli* BL21-DE3 und *E.coli* DH5a wurden mit der durch **"Site** Saturation Mutagenesis" hergestellte DNA transformiert. Dazu wurde zu 50 µl kompetenten Zellen 100 ng der DNA hinzugegeben uns auf Eis für 30 min inkubiert. Anschließend erfolgte ein Hitzeschock für 90 sek. bei 42 °C. Nach 3 min. auf Eis wurden 500 µl SOC-medium hinzugegeben und die Lösung am Anschluss für 45 min bei 350 UPM und 37 °C im Eppendorf Thermomixer inkubiert. Nach erfolgter Inkubation wurde die Zellsuspension bei 13.000 UPM in einer Eppendorf Tischzentrifuge für 30 sek zentrifugiert und das Pellet daraufhin in 100 µl Überstand resuspendiert. Die auf 100 µl eingeengte Zellsuspension wurde auf LB-Aggarplatten (mit 100 µg/ml Ampicillin) ausplattiert und für 16 h bei 37 °C über Kopf inkubiert.

### DNA-Isolierung und Identifizierung der besten Enzymvarianten durch DNA-Sequenzierung

Die DNA des Enzyms, das in den Carboligationsreaktionen den höchsten ee-Werten für (S)-PAC lieferte, wurde ausgehend von der Masterplatte zur Identifizierung der Mutation sequenziert. Dazu wurde zunächst Zellen mit einer Impföse aus der mit Glycerin versetzten Masterplatte 50 mL LB-Medium (+50 µg/ml Ampicillin) überführt und in einem 250 mL Erlenmeyerkolben bei 37 °C inkubiert. Nach 12 h Inkubation wurden 20 mL der Zellsuspension zentrifugiert (4.000 UPM, 5 min, 4 °C). Die DNA der Zellen im Pellet wurde analog zu den Herstellerangaben (Qiagen N.V.) nach der Methode des QIAprep® Spin Miniprep Kit" isoliert. Die Konzentration der DNA wurde dazu auf 100 ng/µl eingestellt und durch die Firma LGC Genomics GmbH sequenziert.

### LB (Lysogeny Broth)-Medium

| | | |
|---|---|---|
| 10 g/l | NaCl | 25 |
| 10 g/l | Peptone | |
| 5 g/l | Yeast extract | |

### Herstellung der Varianten in Form von "ganzen Zellen"

Zur Expression der Enzyme in ganzen Zellen im 1L-Maßstab wurde zunächst Zellen von der mit Glycerin versetzten Masterplatte mit einer Impföse in 50 mL LB-Medium (+100 µg/ml Ampicillin) überführt und in einem 250 mL Erlenmeyerkolben bei 120 UPM und 37 °C inkubiert. Nach 16 h Inkubation wurden die zu 1 L Autoinduktionsmedium 10 mL der Kultur gegeben und in einem 5 L Erlenmeyerkolben für 72 h bei 90 UPM und 20 °C inkubiert. Die Zellen wurden anschließend durch Zentrifugation (4 °C, 6.000 UPM, 30 min) geerntet und bis zur weiteren Verwendung bei -20 °C gelagert.

### Autoinduktionsmedium

| | |
|---|---|
| 12 g/l | Pepton |
| 24 g/l | Hefeextrakt |
| 90 mM | Kaliumphosphat-Puffer (pH 7,5) |
| 0,5 g/l | Glucose |
| 2 g/l | Lactose |
| 0,01 g/l | Ampicillin |
| 6.3 g/l | Glycerin |

### Herstellung der Varianten in Form von isolierten Enzymen

10 g der im 1L-Maßstab kultivierten Zellen wurden mit 25 mL Aufschlusspuffer (50 mM Kaliumphosphat pH 6.5, 100 µM Thiamindiphosphat, 2 mM Magnesiumsulfat), der auf 4 °C gekühlt war, auf Eis resuspendiert. Anschließend wurden die resuspendierten Zellen mittels Ultraschall SD14-Sonotrode (Hielscher Untrasonics GmbH), 4x2 min Beschall mit je 1 min Abkühlung aus Eis) aufgeschlossen. Zur Abtrennung von Zelltrümmern wurde die Lösung zentrifugiert (45 min, 18.000 UPM, 4 °C) und der Überstand ("Zellextrakt") in ein neues Gefäß überführt.
Zur Reinigung der *Ap*PDC-Variante mittels immobilisierte Metallionen-Affinitätschromatographie und Größenausschlusschromatographie wurde ein "ÄKTA™purifier" der Firma "Amersham Bioscience" verwendet, um u.a. die Protein-UV-Absorption (280 nm) sowie die elektrische Leitfähigkeit zu detektieren und die Flussrate einzustellen. Zur Reinigung wurde der hergestellte Zellextrakt (∼25 mL) mit einem Flussrate von 3 mL/min auf eine Säule mit einem Volumen von 60 mL Ni-NTA-Superflow (Qiagen N.V.) aufgetragen, die zuvor mit 180 mL des Auftragspuffer equilibriert wurde. Im Anschluss wurde weiter mit Auftragspuffer in einer Flussrate von 5 ml/min gespült, um nicht gebundene, bzw. sehr schwach an das Säulenmaterial bindende, Proteine zu entfernen. Nachdem die UV-Absorption (280 nm) wieder eine stabile Basisline erreicht hatte, wurde ein Waschpuffer (50 mM Kaliumphosphat pH 6.5, 100 µM Thiamindiphosphat, 2 mM Magnesiumsulfat, 50 mM Imidazol) mit einer Flussrate von 5 mL/min zur Elution von schwach an das Säulenmaterial bindenden Proteinen verwendet. Nach erneut stabiler UV-Absorption (280 nm) wurde zur Elution des Zielproteins ein Elutionspuffer (50 mM Kaliumphosphat pH 6.5, 100 µM Thiamindiphosphat, 2 mM Magnesiumsulfat, 250 mM Imidazol) mit einer Flussrate von 5 mL/min verwendet.
Das Eluat der IMAC wurde zur Umpufferung mit einer Flussrate von 10 mL/min auf eine Größenausschlusschromatographie-Säule (1 L Säulenvolumen, Sephadex-G25, GE-Healthcare) aufgetragen, die zuvor mit 2 L Umpufferungspuffer (10 mM Kaliumphosphat pH 6.5, 100 µM Thiamindiphosphat, 2 mM Magnesiumsulfat) gespült wurde. Die Fraktionen mit erhöhter UV-Absorption (280 nm) wurden vereinigt und in einer Kristallisationsschale eingefroren (-20 °C). Zur Gefriertrocknung wurde an die eingefrorene Proteinlösung für 3 Tage ein Unterdruck von 0.22 mBar angelegt. Das entstandene Pulver hatte einen Proteingehalt von 20 %. Die Reinheit (Anteil des Zielproteins in Bezug auf Fremdproteine) betrug >90 %.

## Patentansprüche

1. Lyase,
bei der die Aminosäure Isoleucin in dem gegenüber dem Wildtyp aus *Acetobacter pasteurianus* veränderten Protein ApPDC-E469G in Position Nr. 468 durch eine Aminosäure ersetzt ist, die weniger raumerfüllend ist als Isoleucin,
**dadurch gekennzeichnet, dass**
sie eine Aminosäuresequenz nach einer der Sequenzen Nr. 1 oder 2 hat.

2. Lyase nach Anspruch 1,
bei der die Aminosäure in Position Nr. 543 zusätzlich durch eine Aminosäure ausgetauscht ist, die eine kleinere Raumerfüllung hat als Tryptophan,
**dadurch gekennzeichnet, dass** sie eine Aminosäuresequenz nach einer der Sequenzen Nr. 10 oder 23 hat.

3. Lyase nach einem der Ansprüche 1 oder 2,
**dadurch gekennzeichnet, dass**
sie eine Aminosäuresequenz nach Sequenz Nr. 23 hat.

4. Desoxyribonukleinsäure, kodierend für eine Lyase nach Anspruch 1,
die für eine Variante des Enzyms *Ap*PDC-E469G kodiert, bei der ausschließlich das Kodon in Position Nr. 1402-1404 durch ein Kodon ausgetauscht ist, welches für eine Aminosäure kodiert, die weniger raumerfüllend ist als Isoleucin,
**dadurch gekennzeichnet, dass**
sie eine Nukleotidsequenz nach einer der Sequenzen Nr. 4 oder 52 hat.

5. Desoxyribonukleinsäure, kodierend für eine Lyase nach einem der Ansprüche 1 bis 3, die für eine Variante des Enzyms *Ap*PDC-E469G kodiert, bei der ausschließlich das Kodon in Position Nr. 1402-1404 durch ein Kodon ausgetauscht ist, welches für eine Aminosäure kodiert, die weniger raumerfüllend ist als Isoleucin und bei der zusätzlich das Kodon in Position Nr. 1627 bis 1629 durch ein Kodon ausgetauscht ist, welches für eine Aminosäure kodiert, die weniger raumerfüllend ist als Tryptophan,
**dadurch gekennzeichnet, dass**
sie eine Nukleotidsequenz nach Sequenz Nr. 48 hat.

6. Vektor,
**dadurch gekennzeichnet,**
**dass** er eine Desoxyribonukleinsäure ausgewählt aus der Gruppe enthaltend die Sequenzen Nr. 4, 48 und 52 beinhaltet.

7. Vektor nach Anspruch 6,
**dadurch gekennzeichnet, dass**
er ein Plasmid ist.

8. Vektor nach Anspruch 7,
**dadurch gekennzeichnet, dass**
die Sequenzen nach Anspruch 7 in einem Leervektor aus der Gruppe pET-20b(+), pET-21a-d(+), pET-22b(+), pET-23a-d(+), pET-24a-d(+), pET-25b(+), pET-26b(+), pET-27b(+), pET-28a(+), pET-29a-c(+), pET-30a-c(+), pET-31 b(+), pET-34b(+), pET-35b(+), pET-36b(+), pET-37b(+), pET-38b(+) ligiert sind.

9. Verfahren zur Herstellung von (S)-Phenylacetylcarbinol, bei dem Benzaldehyd mit Pyruvat oder mit Acetaldehyd nach Formel (1) umgesetzt wird, 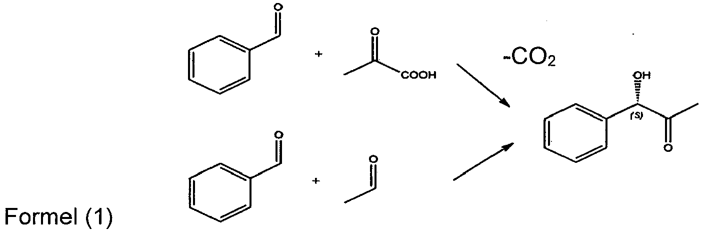 **dadurch gekennzeichnet, dass**
die Umsetzung mit einer Lyase gemäß einer Aminosäuresequenz ausgewählt aus der Gruppe enthaltend die Sequenzen Nr. 1, 2, 10 und 23 durchgeführt wird.

10. Verfahren nach Anspruch 9,
**dadurch gekennzeichnet, dass**
die Umsetzung in *vivo* durchgeführt wird.

11. Verfahren nach einem der Ansprüche 9 oder 10,
**dadurch gekennzeichnet, dass**
die Produktion von (*S*)-Phenylacetylcarbinol in einem Produktionsorganismus aus der Gruppe von *E*.*coli*, ein *Corynebakterium, Corynebakterium glutamicum,* oder einer Hefe, *Saccheromycies cerevisiae* durchgeführt wird.

12. Verfahren nach einem der Ansprüche 9 bis 11,
**dadurch gekennzeichnet, dass**
die Organismen mit mindestens einer DNA ausgewählt aus der Gruppe enthaltend die Sequenzen Nr. 4, Nr. 48 und Nr. 52 transformiert werden und/oder die DNA ausgewählt aus der Gruppe enthaltend die Sequenzen Nr. 4, Nr. 48 und Nr. 52 in das Genom integriert wird.

13. Verfahren nach einem der Ansprüche 9 bis 12,
**dadurch gekennzeichnet, dass**
für die Transformation ein Vektor nach einem der Ansprüche 6 bis 8 eingesetzt wird.

14. Verfahren nach Anspruch 9,
**dadurch gekennzeichnet, dass**
die Umsetzung in *vitro* mit Rohextrakt aus einem Produktionsorganismus oder isoliertem Enzym mit einer Aminosäuresequenz ausgewählt aus der Gruppe enthaltend die Sequenzen Nr. 1, 2, 10 und 23 durchgeführt wird.

## Claims

1. A lyase, wherein the amino acid isoleucine in the protein *Ap*PDC-E469G, which is modified compared to the wild type from *Acetobacterpasteurianus,* in position no. 468 is replaced by an amino acid which is less space-filling than isoleucine,
**characterized in that**
it has an amino acid sequence according to one of the sequences no. 1 or 2.

2. The lyase according to claim 1,
wherein the amino acid in position no. 543 is also replaced by an amino acid which has less space-filling than tryptophan,
**characterized in that** it has an amino acid sequence according to one of the sequences no. 10 or 23.

3. The lyase according to any one of claims 1 or 2,
**characterized in that**
it has an amino acid sequence according to sequence no. 23.

4. Deoxyribonucleic acid, coding for a lyase according to claim 1,
which codes for a variant of the enzyme *Ap*PDC-E469G, wherein exclusively the codon in position nos. 1402-1404 is replaced by a codon which codes for an amino acid that is less space-filling than isoleucine,
**characterized in that**
it has a nucleotide sequence according to one of the sequences no. 4 or 52.

5. The deoxyribonucleic acid, coding for a lyase according to any one of claims 1 to 3, which codes for a variant of the enzyme ApPDC-E469G, wherein exclusively the codon in position nos. 1402-1404 is replaced by a codon, which codes for an amino acid which is less space-filling than isoleucine and wherein the codon in position nos. 1627 to 1629 is also replaced by a codon, which codes for an amino acid, which is less space-filling than tryptophan,
**characterized in that**
it has a nucleotide sequence according to sequence no. 48.

6. A vector,
**characterized in**
**that** it comprises a deoxyribonucleic acid selected from the group containing the sequences nos. 4, 48 and 52.

7. The vector according to claim 6,
**characterized in that**
it is a plasmid.

8. The vector according to claim 7,
**characterized in that**
the sequences according to claim 7 are ligated in an empty vector from the group pET-20b(+), pET-21a-d(+), pET-22b(+), pET-23a-d(+), pET-24a-d(+), pET-25b(+), pET-26b(+), pET-27b(+), pET-28a(+), pET-29a-c(+), pET-30a-, c(+), pET-31 b(+), pET-34b(+), pET-35b(+), pET-36b(+), pET-37b(+), pET-38b(+).

9. A method for the production of (S)-phenylacetylcarbinol, wherein benzaldehyde is reacted with pyruvate or with acetaldehyde according to formula (1), 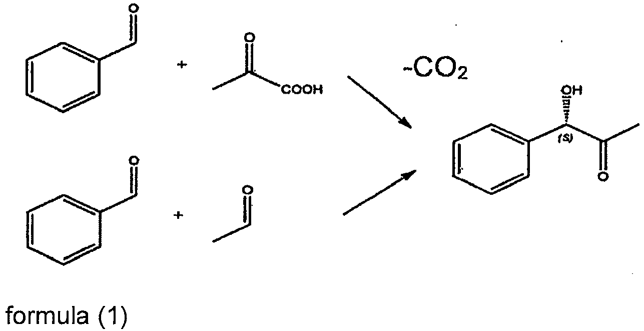 **characterized in that**
the reaction is carried out with a lyase according to an amino acid sequence selected from the group containing the sequences nos. 1, 2, 10 and 23.

10. The method according to claim 9,
**characterized in that**
the reaction is carried out in *vivo.*

11. The method according to any one of claims 9 or 10,
**characterized in that**
the production of (S)-phenylacetylcarbinol is carried out in a production organism from the group of *E.coli,* a *Corynebacterium, Corynebacterium glutamicum,* or a yeast, *Saccheromycies cerevisiae.*

12. The method according to any one of claims 9 to 11,
**characterized in that**
the organisms are transformed with at least one DNA selected from the group containing the sequences no. 4, no. 48 and no. 52, and/or the DNA selected from the group containing the sequences no. 4, no. 48 and no. 52 is integrated into the genome.

13. The method according to any one of claims 9 to 12,
**characterized in that**
a vector according to any one of claims 6 to 8 is used for the transformation.

14. The method according to claim 9,
**characterized in that**
the reaction is carried out in *vitro* with crude extract from a production organism or isolated enzyme with an amino acid sequence selected from the group containing the sequences nos. 1, 2, 10 and 23.

## Revendications

1. Lyase,
dans laquelle l'acide aminé isoleucine dans la protéine ApPDC-E469G modifiée par rapport au type sauvage d'*Acetobacter pasteurianus* est remplacé en la position No. 468 par un acide aminé, qui remplit moins d'espace que l'isoleucine,
**caractérisée en ce que**
elle a une séquence d'acides aminés suivant l'une des séquences No. 1 ou 2.

2. Lyase suivant la revendication 1,
dans laquelle l'acide aminé en la position No. 543 est échangé avec un acide aminé, qui remplit moins d'espace que le tryptophane,
**caractérisée en ce qu'**elle a une séquence d'acides aminés suivant l'une des séquences No. 10 ou 23.

3. Lyase suivant l'une des revendications 1 ou 2,
**caractérisée en ce que**
elle a une séquence d'acides aminés suivant la séquence No. 23.

4. Acide désoxyribonucléique codant pour une lyase suivant la revendication 1,
qui code pour une variante de l'enzyme ApPDC-E469G, dans lequel exclusivement le codon en la position No. 1402 à 1404 est échangé avec un codon, qui code pour un acide aminé, qui remplit moins d'espace que l'isoleucine,
**caractérisé en ce que**
il a une séquence de nucléotides suivant l'une des séquences No. 4 ou 52.

5. Acide désoxyribonucléique codant pour une lyase suivant l'une des revendications 1 à 3,
qui code pour une variante de l'enzyme ApPDC-E469G, dans lequel exclusivement le codon en la position No. 1402 à 1404 est échangé avec un codon, qui code pour un acide aminé, qui remplit moins d'espace que l'isoleucine et dans lequel supplémentairement le codon en la position No. 1627 à 1629 est échangé avec un codon qui code pour un acide aminé, qui remplit moins d'espace que le tryptophane,
**caractérisé en ce que**
il a une séquence de nucléotides suivant la séquence No. 48.

6. Vecteur,
**caractérisé**
**en ce qu'**il contient un acide désoxyribonucléique choisi dans le groupe contenant les séquences No. 4, 48 et 52.

7. Vecteur suivant la revendication 6,
**caractérisé en ce que**
c'est un plasmide.

8. Vecteur suivant la revendication 7,
**caractérisé en ce que**
les séquences suivant la revendication 7 sont ligaturées en un vecteur vide choisi dans le groupe pET-20b(+), pET-21ad(+), pET-22b(+), pET-23a-d(+), pET-24a-d(+), pET-25b(+), pET-26b(+), pET-27b(+), pET-28a(+), pET-29a-c(+), pET-30a-c(+), pET-31b(+), pET-34b(+), pET-35b(+), pET-36b(+), pET-37b(+), pEt-38b(+).

9. Procédé de préparation de (*S*)-phénylacétylcarbinol, dans lequel on fait réagir le benzaldéhyde sur un pyruvate ou l'acétaldéhyde de formule (1), 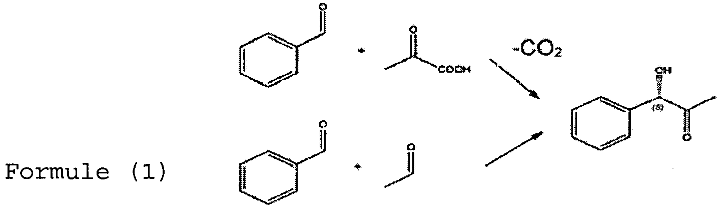 **caractérisé en ce que**
l'on effectue la réaction avec une lyase suivant la séquence d'acides aminés du groupe contenant les séquences No. 1, 2, 10 et 23.

10. Procédé suivant la revendication 9,
**caractérisé en ce que**
l'on effectue la réaction *in vivo.*

11. Procédé suivant l'une des revendications 9 ou 10,
**caractérisé en ce que**
l'on effectue la production de (*S*)-phénylacétylcarbinol dans un organisme de production du groupe de *E. coli*, un *corynebakterium, corynebakterium glutamicum,* ou une levure, *saccheromycies cerevisiae.*

12. Procédé suivant l'une des revendications 9 à 11,
**caractérisé en ce que**
l'on transforme les organismes ayant au moins un ADN choisi dans le groupe contenant les séquences No. 4, No. 48 et No. 52 et/ou on intègre au génome l'ADN choisi dans le groupe contenant les séquences No. 4, No. 48 et No. 52.

13. Procédé suivant l'une des revendications 9 à 12,
**caractérisé en ce que**
l'on utilise pour la transformation un vecteur suivant l'une des revendications 6 à 8.

14. Procédé suivant la revendication 9,
**caractérisé en ce que**
l'on effectue la réaction *in vitro* avec un extrait brut d'un organisme de production ou un enzyme isolé ayant une séquence d'acides aminés choisie dans le groupe contenant les séquences No. 1, 2, 10 et 23.
